(19) Europäisches Patentamt
European Patent Office
Office européen des brevets

(11) **EP 2 849 695 B1**

(12) **EUROPÄISCHE PATENTSCHRIFT**

(45) Veröffentlichungstag und Bekanntmachung des Hinweises auf die Patenterteilung:
**13.04.2016 Patentblatt 2016/15**

(21) Anmeldenummer: **13719840.4**

(22) Anmeldetag: **30.04.2013**

(51) Int Cl.:
***A61F 13/49*** *(2006.01)*        ***A61F 13/496*** *(2006.01)*

(86) Internationale Anmeldenummer:
**PCT/EP2013/058978**

(87) Internationale Veröffentlichungsnummer:
**WO 2013/171067 (21.11.2013 Gazette 2013/47)**

(54) **INKONTINENZARTIKEL IN HÖSCHENFORM**

INCONTINENCE ARTICLE IN THE FORM OF BRIEFS

ARTICLE POUR INCONTINENCE EN FORME DE CULOTTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.05.2012 DE 102012208394**

(43) Veröffentlichungstag der Anmeldung:
**25.03.2015 Patentblatt 2015/13**

(73) Patentinhaber: **Paul Hartmann AG**
**89522 Heidenheim (DE)**

(72) Erfinder:
• **GASSNER, Oliver**
  **50672 Köln (DE)**
• **BEYRLE, Andreas**
  **89564 Nattheim (DE)**
• **KESSELMEIER, Ruediger**
  **89542 Hebrechtingen (DE)**
• **MALOWANIEC, Krzysztof**
  **89522 Heidenheim (DE)**

(74) Vertreter: **DREISS Patentanwälte PartG mbB**
**Friedrichstrasse 6**
**70174 Stuttgart (DE)**

(56) Entgegenhaltungen:
**WO-A1-2011/098226    WO-A1-2011/105475**
**JP-A- H1 136 103    JP-A- 2006 204 673**
**JP-A- 2007 082 890    JP-A- 2008 253 289**
**US-A1- 2004 186 453    US-A1- 2005 107 764**
**US-A1- 2006 025 746    US-A1- 2009 178 755**

EP 2 849 695 B1

**Beschreibung**

Die Erfindung betrifft einen Inkontinenzartikel in Höschenform für die Aufnahme von Körperausscheidungen mit einem vorderen Bauchabschnitt und einem hinteren Rückenabschnitt, die von separaten und in einer Längsrichtung entlang einer Längsmittelachse voneinander beabstandeten Komponenten gebildet sind, jedoch zur Bildung eines in Quer- oder Hüftumfangsrichtung durchgehenden Bauch- und Rückenbands mit einer in Hüftumfangsrichtung geschlossenen Hüftöffnung an beidseitigen Seitennahtbereichen herstellerseitig miteinander verbunden sind, wobei jeweils eine in einer Längsrichtung erstreckte Seitennaht gebildet ist, und mit einem einen Absorptionskörper aufweisenden Schrittabschnitt, der sich in der Längsrichtung zwischen Bauchabschnitt und Rückenabschnitt erstreckt und mit seiner körperabgewandten Seite an den Bauchabschnitt und an den Rückenabschnitt in einem jeweiligen Überlappungsbereich unlösbar angefügt ist, wobei der Bauchabschnitt, der Rückenabschnitt und der Schrittabschnitt gemeinsam Beinöffnungen des Inkontinenzartikels begrenzen, wobei in dem Bauchabschnitt und dem Rückenabschnitt erste Elastifizierungsmittel vorgesehen sind, die sich in einem Abstand voneinander und parallel zueinander in Quer- oder Hüftumfangsrichtung erstrecken und so den Bauchabschnitt und den Rückenabschnitt flächenhaft elastifizieren, wobei in einem schrittseitigen und den Beinöffnungen zugewandten Bereich des Bauchabschnitts und des Rückenabschnitts zweite Elastifizierungsmittel vorgesehen sind, die sich ausgehend von den beiden Seitennahtbereichen in Richtung auf eine Längsmittelachse des Inkontinenzartikels erstrecken und entlang ihrer Erstreckung in Richtung auf die Längsmittelachse des Inkontinenzartikels bogenförmig sich auffächernd mit zunehmendem Abstand voneinander verlaufen und sich bis in den Überlappungsbereich von Schrittabschnitt und Bauchabschnitt (im folgenden auch vorderer Überlappungsbereich genannt) bzw. von Schrittabschnitt und Rückenabschnitt (im folgenden auch hinterer Überlappungsbereich genannt) erstrecken, wobei sie dort ihrer elastifizierenden Wirkung benommen sein können, insbesondere geschnitten sein können.

Es handelt sich also um einen dreikomponentigen Inkontinenzartikel, wobei der Bauchabschnitt, der Rückenabschnitt und der Schrittabschnitt diese drei Komponenten bilden. Der Bauchabschnitt und der Rückenabschnitt sowie der Schrittabschnitt werden als voneinander separate Komponenten einer Herstellungsvorrichtung zugeführt bzw. darin gefördert. Dabei sind die Komponenten typischerweise in einer jeweiligen Förderebene in einem flach oder eben ausgebreiteten Zustand geführt. Dabei werden der Bauchabschnitt und der Rückenabschnitt in der späteren Querrichtung des Inkontinenzartikels gefördert; sie sind dabei in der späteren Längsrichtung des Inkontinenzartikels voneinander beabstandet geführt. Somit verläuft die spätere Quer- oder Hüftumfangsrichtung des Inkontinenzartikels in der Maschinenrichtung der Herstellungsvorrichtung. Der vorgenannte Abstand zwischen Bauchabschnitt und Rückenabschnitt wird dann durch Aufbringen des Schrittabschnitts als dritter Komponente gewissermaßen überbrückt, wobei ein Überlappungsbereich zwischen Schrittabschnitt und Bauchabschnitt und zwischen Schrittabschnitt und Rückenabschnitt gebildet wird, wobei die drei Komponenten in dem jeweiligen Überlappungsbereich unlösbar miteinander gefügt werden. Schließlich werden der Bauchabschnitt und der Rückenabschnitt wie vorstehend erwähnt an beidseitigen Seitennahtbereichen miteinander verbunden, wobei die Erstreckung der jeweiligen Seitennaht in Längsrichtung 100-170 mm beträgt. Ein derartiger Inkontinenzartikel ist beispielsweise bekannt aus DE 10 2007 055 628 A1.

Inkontinenzartikel in Höschenform unterscheiden sich prinzipiell von öffenbaren und schließbaren Inkontinenzartikeln in üblicher Windelform dadurch, dass durch die Höschenform der Hüftumfang schon vorgegeben ist und die Anpassung an unterschiedliche Körpergrößen ausgehend von einer Anzahl von Grundgrößen durch eine elastische Dehnbarkeit des Artikels erreicht wird. Hierfür werden in der Regel Elastifizierungsmittel, insbesondere in Form von Bändern oder Fäden, häufig als Lycra-Fäden bezeichnet, in vorgedehntem Zustand (Stretch-Bond-Verfahren) mit Chassismaterialien des Inkontinenzartikels verbunden, das heißt, sie werden in vorgedehntem Zustand an den Chassismaterialien z.B. mittels Kleber fixiert. Infolge ihrer Vorspannung raffen diese Elastifizierungsmittel die Chassismaterialien zusammen und bilden dabei Fältelungen, die typischerweise quer zur Vorspannungsrichtung der Elastifizierungsmittel, hier also in Längsrichtung des Artikels, verlaufen. Der Inkontinenzartikel bzw. die elastifizierten Chassismaterialien des Inkontinenzartikels lassen sich dann wieder elastisch dehnen, wenn der Inkontinenzartikel wie ein Höschen an den Benutzer angelegt wird. Die Chassismaterialien selbst sind hingegen vorzugsweise undehnbar und lassen sich daher in wohldefinierter Weise in der Förderebene in einem flach oder eben ausgebreiteten Zustand führen, so dass die Elastifizierungsmittel dann mit definierter Vorspannung angefügt werden können. WO 2011/098226 A1, US 2009/0178755 A1 offenbaren gattungsgemäße Inkontinenzartikel in Höschenform. US 2005/0107764 A1 zeigt ebenfalls einen Inkontinenzartikel in Höschenform der hier in Rede stehenden dreikomponentigen Bauform mit einer Vielzahl von in Querrichtung und parallel zueinander erstreckten Elastifizierungsmitteln. Nicht gattungsgemäße Inkontinenzartikel in Höschenform, bei denen ein sich über die gesamte Fläche erstreckendes Chassis vorgesehen ist, sind beschrieben in JP 11036103 A, US 2006/0025746 A1, JP 2006-204673 A, US 2004/0186453 A1, JP 2008-253289 A, JP 2007-82890 A und WO 2011/105475 A1.

Bei vorbekannten Inkontinenzartikeln der hier in Rede stehenden Art besteht die Tendenz, den Bauchabschnitt und den Rückenabschnitt und damit das von ihnen gebildete in Hüftumfangsrichtung durchgehende Bauch- bzw. Rückenband in Körperlängsrichtung verhältnismäßig ausladend auszubilden, so dass der Hüftbund oder Hüftrand verhältnismäßig hoch am Benutzer zu liegen kommt. Hierdurch soll eine verhältnismäßig große körperkontaktierende Fläche des über seine Erstreckung flächenhaft elastifizierten Bauch- und Rückenbands geschaffen werden, durch die ja allein der Sitz der Inkontinenzartikels am Körper des Benutzers bestimmt und aufrechterhalten wird. Je größer die körperkontaktierende und elastifizierte Fläche von Bauchabschnitt und Rückenabschnitt ist, umso größer sind die den Inkontinenzartikel am Körper haltenden Kräfte. Es muss schließlich sichergestellt werden, dass der Inkontinenzartikel beim üblichen bestimmungsgemäßen Gebrauch nicht nach unten rutscht.

Im Ausgangspunkt der vorliegenden Erfindung wurde erkannt, dass ein breites Bauch- und Rückenband, d.h. ein hoch liegender Hüftrand oder Hüftbund, gerade bei mobilen Benutzern und in sehr verstärktem Maße bei Benutzern mit Bauchansatz mit dem Problem verbunden ist, dass sich der Hüftrandbereich des Inkontinenzartikels nach außen überfaltet. Hierbei kann es zu einem Überfalten oder Überrollen von vielen Zentimetern in Längsrichtung kommen, was dann mit einer dramatischen Reduzierung der körperkontaktierenden Fläche verbunden ist, wonach der sichere Sitz des Inkontinenzartikels gefährdet wird. Dessen ungeachtet führt dies zu einem starken lokalen Anstieg von in Umfangsrichtung wirkenden einschnürenden Kräften, da eine Mehrzahl von Elastifizierungsmitteln gewissermaßen auf einer Hüftumfangslinie zu liegen kommen. Dies führt jedoch zu einem unangenehmen Traggefühl bis hin zu schweren Hautreizungen, nicht aber zu einer Verbesserung des Sitzes des Hygieneartikels.

Auch eine starke Reduzierung der Breite bzw. Längserstreckung des Bauch- und Rückenbands vermag nicht zu befriedigenden Tragesituationen zu führen, obschon hierdurch das Problem des nach außen Umfaltens des Hüftrands reduziert werden kann. Es entstehen aber gleichwohl einschnürende Kräfte, und die Elastifizierung des Bauch- und Rückenbands kann nicht zufriedenstellend gelöst werden.

Ausgehend von diesen bereits in erfinderischer Weise gewonnenen Erkenntnissen liegt der vorliegenden Erfindung die Aufgabe zugrunde, die Passform, den Sitz und damit den Tragkomfort eines hier in Rede stehenden Inkontinenzartikels in wirtschaftlich vertretbarer Weise weiter zu verbessern, wobei den vorstehend geschilderten Problemen wirksam begegnet werden soll.

Diese Aufgabe wird bei einem Inkontinenzartikel der genannten Art erfindungsgemäß dadurch gelöst, dass das Verhältnis (L2/L1) der Erstreckung (L2) der jeweiligen Seitennaht in Längsrichtung zur Erstreckung (L1) des Inkontinenzartikels zwischen Hüftrand und einer Quermittelachse höchstens 0,42 beträgt, und dass im Bauchabschnitt und im Rückenabschnitt das Verhältnis (L4/L1) des Abstands (L4) des äußersten hüftzugewandten ersten Elastifizierungsmittels in Längsrichtung vom innersten schrittzugewandten ersten Elastifizierungsmittel zur Erstreckung (L1) des Inkontinenzartikels zwischen Hüftrand und der Quermittelachse höchstens 0,3 beträgt, und dass die ersten Elastifizierungsmittel einen Abstand voneinander haben, der wenigstens 20 % größer ist als der im Seitennahtbereich bestimmte Abstand der zweiten Elastifizierungsmittel voneinander, und dass die ersten Elastifizierungsmittel eine um wenigstens 20 % größere Fadenstärke haben als die zweiten Elastifizierungsmittel und mit einer um wenigstens 10 % größeren Vorspannung im Bauchabschnitt und im Rückenabschnitt fixiert sind als die zweiten Elastifizierungsmittel.

Erfindungsgemäß wird also vorgeschlagen, das in Quer- oder Hüftumfangsrichtung durchgehende Bauch- und Rückenband, welches von dem Bauchabschnitt und dem Rückenabschnitt gebildet wird, schmäler zu gestalten, so dass der Hüftrand beim Benutzer in Körperlängsrichtung des stehenden Benutzers tiefer zu liegen kommt. Zum Ausgleich der hierdurch verringerten flächenhaften Erstreckung des Bauchabschnitts und des Rückenabschnitts wird jedoch nicht etwa die Zahl der Elastifizierungsmittel vergrößert, sondern es wird im Gegenteil der Abstand der ersten Elastifizierungsmittel voneinander größer gewählt als bei den zweiten Elastifizierungsmitteln. Auf diese Weise wird die Anzahl der ersten Elastifizierungsmittel wesentlich reduziert, was den Hygieneartikel zudem hinsichtlich seiner Wirtschaftlichkeit verbessert. Es wurde erfindungsgemäß weiter erkannt, dass diese Verringerung der flächenhaften Erstreckung und die Reduzierung der Anzahl der Elastifizierungsmittel durch eine Vergrößerung des Abstands der ersten Elastifizierungsmittel voneinander nicht zu Nachteilen führt, sofern die Rückstellkraft eines jeweiligen ersten Elastifizierungsmittels erhöht wird, indem für die ersten Elastifizierungsmittel eine größere Fadenstärke und eine stärkere Vorspannung beim Einbringen und Fixieren der ersten Elastifizierungsmittel eingesetzt wird. In bevorzugter Weise wird sowohl eine größere Fadenstärke als auch eine größere Vorspannung gewählt. Die Vorspannung ist dabei definiert als der Dehnungsgrad eines gedehnten Elastifizierungsmittels gegenüber dem ungedehnten/relaxierten Ausgangszustand des Elastifizierungsmittels im Zustand des Aufbringens und Fixierens der Elastifizierungsmittel in der Herstellungsmaschine. Der Dehnungsgrad errechnet sich also als Verhältnis der gedehnten Länge L' (=Ausgangslänge L + ΔL) zur Ausgangslänge L, also L'/L.

Die Fadenstärke der Elastifizierungsmittel wird in der Einheit dtex angegeben (1 dtex = 1g/10000 m). Die Fadenstärke wird entsprechend den Prüfvorschriften BISFA, The International Bureau for the Standardization of man-made Fibres, Test methods for bare elastane yarns, Ausgabe 1998, Kapitel 5: "Determination of linear density" (Bestimmung der linearen Dichte) bestimmt. Die Fadenstärke oder lineare Dichte wird durch Bestimmung der Masse eines Prüfling einer bekannten Fadenlänge von 1000 mm (abgetrennt unter einer Standardvorspannung von 0,1 ± 0,01 mN/tex) nach einer Konditionierung unter Standardbedingungen (23°C ± 2°C, 50% ± 5% relative Luftfeuchtigkeit) im entspannten Zustand bestimmt.

Die Fadenstärke (in dtex) errechnet sich aus dem Quotienten der Masse (in g) durch die Länge des Abschnitts (in m) multipliziert mit dem Faktor 10000.

Es werden hierfür fünf Abschnitte mit jeweils etwa 1300 mm Länge des fadenförmigen oder bandförmigen Elastifizierungsmittels unter geringstmöglicher Spannung von einer Rolle oder Packung abgeschnitten, und zwar in unregelmäßigen Abständen von wenigstens 2 m. Diese fünf Abschnitte werden spannungslos relaxiert und unter den Standardbedingungen für wenigstens vier Stunden ruhen gelassen. Sodann wird ein Prüfling von 1000 mm ± 1 mm Länge von dem jeweiligen 1300 mm langen Abschnitt abgeschnitten, während der betreffende Abschnitt unter einer Vorspannung von 0,1 mN/tex gehalten wird. Diese abgetrennten Prüflinge von 1000 mm Länge werden auf eine Genauigkeit von ± 1 % ihrer erwarteten Masse gewogen. Für jeden Prüfling wird dessen Fadenstärke durch Multiplikation der jeweiligen Masse mit dem Faktor 10000 in dtex erhalten. Aus den fünf Prüflingen wird der arithmetische Mittelwert berechnet, der als Fadenstärke für die hier in Rede stehenden Zwecke verwendet wird.

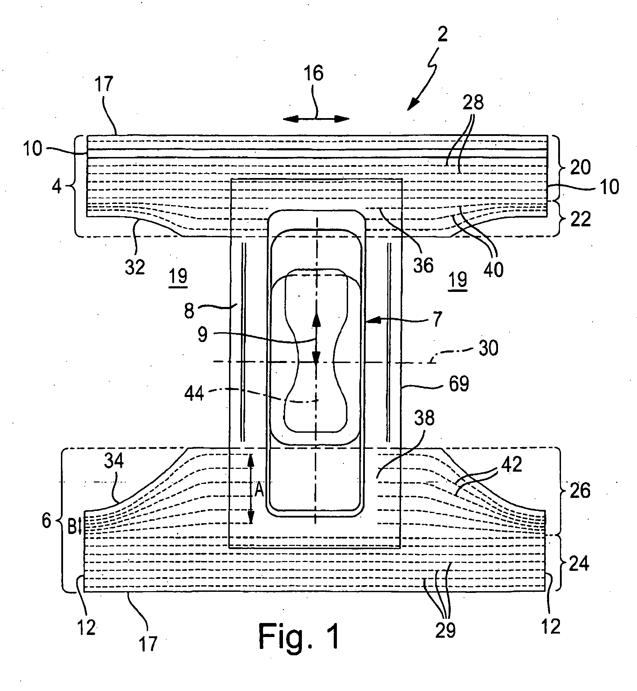

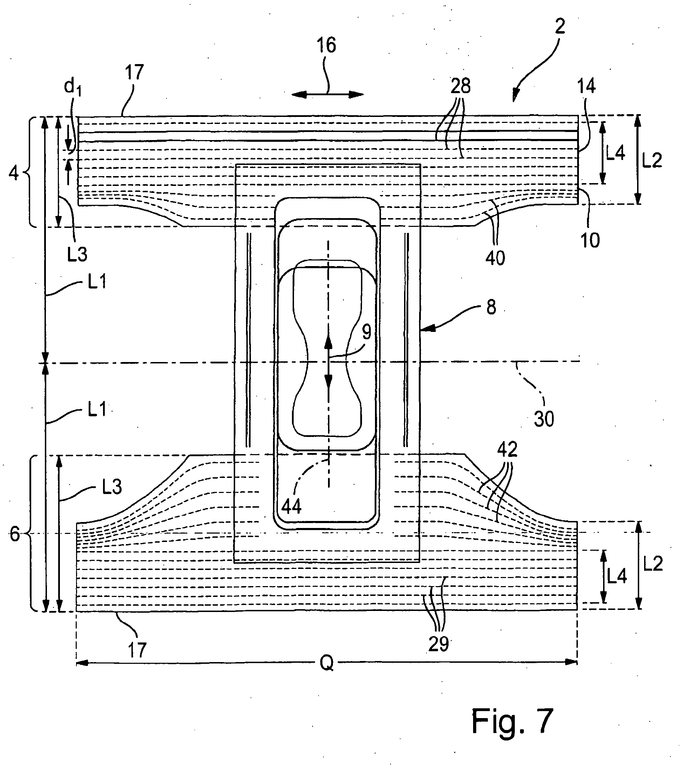

Zur Bestimmung der Erstreckung L1 des Inkontinenzartikels zwischen Hüftrand und der Quermittelachse wird die Quermittelachse so definiert, dass sie die Längserstreckung des Inkontinenzartikels im ausgedehnten flachgelegten Zustand während der Führung der Flachmaterialbahnen in der Herstellungsmaschine in zwei gleiche Teilstrecken teilt (Figur 7). Die Gesamtlänge der Windel beträgt also 2 x L1. Auch alle übrigen hier erwähnten Abmessungen sind auf diesen auch in Figur 1 dargestellten flach ausgedehnten Zustand der Flachmaterialien bezogen.

Die Längserstreckung L1 des hier in Rede stehenden Inkontinenzartikels beträgt bei typischen Größen 320 bis 450 mm, insbesondere 330 bis 440 mm und weiter insbesondere 340 bis 430 mm.

Wie erwähnt verlaufen die ersten Elastifizierungsmittel im Wesentlichen in Quer- oder Hüftumfangsrichtung. Die zweiten Elastifizierungsmittel erstrecken sich - wie erwähnt - ebenfalls ausgehend von einem jeweiligen Seitennahtbereich in Richtung auf die Längsmittelachse; sie fächern sich dabei aber auf und verlaufen mehr oder weniger bogenförmig, wobei sich die Bogenform zunächst in Richtung auf die Quermittelachse hin krümmt, so wie dies gut aus Figur 1 ersichtlich ist. Der Abstand der zweiten Elastifizierungsmittel voneinander in einem jeweiligen Seitennahtbereich beträgt etwa 3 bis 8 mm. Weiter innen in Richtung auf die Längsmittelachse zu und insbesondere im Bereich eines Absorptionskörperrands liegt der Abstand der zweiten Elastifizierungsmittel voneinander zwischen etwa 7 und 35 mm, insbesondere zwischen 12 und 30 mm.

Vorteilhafterweise verlaufen die zweiten Elastifizierungsmittel im Überlappungsbereich zumindest im Bereich der Längsmittelachse in Querrichtung und vorzugsweise auch parallel zueinander.

Diese Auffächerung der zweiten Elastifizierungsmittel lässt sich auch quantitativ näher bezeichnen. Beispielsweise haben die in Figur 1 dargestellten zweiten Elastifizierungsmittel des Rückenabschnitts in den Seitennahtbereichen einen minimalen Abstand von 3 bis 8 mm voneinander (Abstand von unmittelbar nebeneinander liegenden Elastifizierungsmitteln) und an einem Absorptionskörperrand oder einem Längsrand des Schrittabschnitts einen maximalen Abstand (Abstand von unmittelbar nebeneinander liegenden Elastifizierungsmitteln) von 7 bis 35 mm voneinander. Es lässt sich ein Auffächerungsgrad F wie folgt definieren:

$$F = (A-B)/B * 100\%.$$

Dieser Auffächerungsgrad kann vorteilhafterweise zwischen 50 und 900 %, insbesondere zwischen 100 und 700 % und weiter insbesondere zwischen 150 bis 550 % liegen. Er ist im Rückenabschnitt vorteilhafterweise größer als im Bauchabschnitt. Dabei sind die Größen A und B definiert als der Abstand des in Längsrichtung äußersten zweiten Elastifizierungsmittels von dem in Längsrichtung innersten zweiten Elastifizierungsmittel (also nicht der Abstand von unmittelbar nebeneinander liegenden Elastifizierungsmitteln), und zwar A als der maximale Abstand, insbesondere am Längsrand des Schrittabschnitts, und B als der minimale Abstand, insbesondere im Seitennahtbereich.

Es erweist sich weiter als vorteilhaft, wenn das Verhältnis (L2/L1) der Erstreckung (L2) der jeweiligen Seitennaht in Längsrichtung zur Erstreckung (L1) des Inkontinenzartikels zwischen Hüftrand und einer Quermittelachse höchstens 0,4, insbesondere höchstens 0,39, insbesondere höchstens 0,38 und weiter insbesondere wenigstens 0,20, weiter insbesondere wenigstens 0,25, weiter insbesondere wenigstens 0,30 beträgt. Es wurde erfindungsgemäß festgestellt, dass innerhalb dieser Bereichsgrenzen eine gut funktionierende Konfiguration des Inkontinenzartikels im Hinblick auf die hier in Rede stehenden Gesichtspunkte geschaffen wird. - Auch erweist es sich als vorteilhaft, wenn im Bauchabschnitt und im Rückenabschnitt das Verhältnis (L4/L1) des Abstands (L4) des äußersten hüftzugewandten ersten Elastifizierungsmittels in Längsrichtung vom innersten schrittzugewandten ersten Elastifizierungsmittel zur Erstreckung (L1) des Inkontinenzartikels zwischen Hüftrand und der Quermittelachse höchstens 0,29 und insbesondere wenigstens 0,12, insbesondere wenigstens 0,15, insbesondere wenigstens 0,18 beträgt.

Als absolute Größe für den Abstand L4 des äußersten hüftzugewandten ersten Elastifizierungsmittels in Längsrichtung vom innersten schrittzugewandten ersten Elastifizierungsmittel erweist es sich sowohl im Bauchabschnitt als auch im Rückenabschnitt als vorteilhaft, wenn dieser Abstand L4 höchstens 120 mm, insbesondere höchstens 110 mm, insbesondere höchstens 100 mm, insbesondere höchstens 90 mm und insbesondere wenigstens 60 mm, insbesondere wenigstens 70 mm, insbesondere 70 - 110 mm, insbesondere 70-100 mm, insbesondere 70 - 90 mm beträgt.

Aufgrund der Beinöffnungen besitzt der Bauchabschnitt und der Rückenabschnitt nicht etwa im Seitennahtbereich, sondern zur Mitte des Inkontinenzartikels hin, wo der Schrittabschnitt vorgesehen ist, seine maximale Erstreckung in Längsrichtung. Es erweist sich als vorteilhaft, wenn die maximale Erstreckung (L3B) des Bauchabschnitts in Längsrichtung 135 - 260 mm, insbesondere 145-250 mm beträgt und/oder die maximale Erstreckung (L3R) des Rückenabschnitts in Längsrichtung 200-320 mm, insbesondere 210-310 mm beträgt.

Es wurde eingangs darauf hingewiesen, dass das dreikomponentige Konzept des Hygieneartikels bestehend aus Bauchabschnitt, Rückenabschnitt und diese verbindenden Schrittabschnitt impliziert, dass die den Bauchabschnitt und den Rückenabschnitt bildenden Bahnen in Längsrichtung voneinander beabstandet sind. Es erweist sich hierbei als vorteilhaft, wenn ein minimaler Abstand des Bauchabschnitts und des Rückenabschnitts in Längsrichtung voneinander 250 -400 mm, insbesondere 270 - 400 mm, weiter insbesondere 300 - 390 mm beträgt.

Des Weiteren wird vorgeschlagen, dass das Verhältnis (L2/Q) der Erstreckung (L2) der jeweiligen Seitennaht in Längsrichtung zur Erstreckung (Q) des Bauchabschnitts bzw. des Rückenabschnitts in Querrichtung höchstens 0,22, insbesondere höchstens 0,20, insbesondere wenigstens 0,10, insbesondere wenigstens 0,14 beträgt.

In ähnlicher Weise lässt sich die Größe L4 auf die Quererstreckung des Bauchabschnitts bzw. des Rückenabschnitts beziehen. Es erweist sich hier als vorteilhaft, wenn im Bauchabschnitt und im Rückenabschnitt das Verhältnis (L4/Q) des Abstands (L4) des äußersten hüftzugewandten ersten Elastifizierungsmittels in Längsrichtung vom innersten schrittzugewandten ersten Elastifizierungsmittel zur Erstreckung (Q) des Bauchabschnitts bzw. des Rückenabschnitts in Querrichtung höchstens 0,15, insbesondere höchstens 0,14, insbesondere höchstens 0,13 und insbesondere wenigstens 0,05 und weiter insbesondere wenigstens 0,08 und weiter insbesondere wenigstens 0,10 beträgt.

Als ganz besonders vorteilhaft erweist es sich insbesondere im Hinblick auf eine wirtschaftliche Herstellbarkeit des Inkontinenzartikels, wenn die Zahl der Elastifizierungsmittel reduziert werden kann. Insofern wird vorgeschlagen, dass im Bauchabschnitt und/oder im Rückenabschnitt das Verhältnis ($d_1$/L4) des Abstands ($d_1$) der ersten Elastifizierungsmittel in Längsrichtung voneinander zum Abstand (L4) des äußersten hüftzugewandten ersten Elastifizierungsmittels in Längsrichtung vom innersten schrittzugewandten ersten Elastifizierungsmittel zwischen 0,08 und 0,25, insbesondere zwischen 0,09 und 0,20, insbesondere zwischen 0,10 und 0,18 beträgt. In Absolutwerten beträgt der Abstand ($d_1$) der ersten Elastifizierungsmittel in Längsrichtung voneinander wenigstens 8 mm, insbesondere wenigstens 10 mm, insbesondere 10 - 15 mm, insbesondere 11-14 mm, weiter insbesondere 12-13 mm. Vorzugsweise haben die ersten Elastifizierungsmittel denselben Abstand voneinander.

Im Unterschied hierzu beträgt ein minimaler Abstand der zweiten Elastifizierungsmittel voneinander in ihrem Ausgangspunkt in den Seitennahtbereichen 3 bis 8 mm, insbesondere 3 bis 7 mm, insbesondere 3 bis 6 mm.

Es erweist sich weiter als vorteilhaft, wenn die ersten Elastifizierungsmittel einen Abstand voneinander haben, der wenigstens 30 %, insbesondere wenigstens 50 %, insbesondere höchstens 200 %, insbesondere höchstens 180 %, insbesondere höchstens 150 % größer ist als der im Seitennahtbereich bestimmte Abstand der zweiten Elastifizierungsmittel voneinander.

Als erste und/oder zweite Elastifizierungsmittel werden vorzugsweise faden- oder bandförmige Elastifizierungsmittel, wie Gummi- oder Polyetherpolyurethan- oder Polyesterpolyurethanfäden, vorzugsweise Elastikfäden wie Lycra®- oder Spandex®-Fäden eingesetzt.

In Weiterbildung der Erfindung wird vorgeschlagen, dass die ersten Elastifizierungsmittel eine um wenigstens 30 %, insbesondere um wenigstens 50 %, insbesondere um höchstens 150 %, insbesondere um höchstens 130 %, insbesondere um höchstens 100 % größere Fadenstärke haben als die zweiten Elastifizierungsmittel.

Weiter erweist es sich als vorteilhaft, wenn die Fadenstärke der ersten Elastifizierungsmittel, wenigstens 1000 dtex, insbesondere wenigstens 1100 dtex, insbesondere wenigstens 1200 dtex, insbesondere 1200 - 1500 dtex, insbesondere 1200 - 1400 dtex beträgt.

In weiterer Ausbildung der vorliegenden Erfindung wird vorgeschlagen, dass die Fadenstärke der zweiten Elastifizierungsmittel 500 - 1100 dtex, insbesondere 600 - 1000 dtex, insbesondere 700 - 900 dtex beträgt.

In weiterer Ausbildung der vorliegenden Erfindung wird vorgeschlagen, dass die ersten Elastifizierungsmittel mit einer um wenigstens 20 %, insbesondere um wenigstens 30 %, insbesondere um höchstens 100 %, insbesondere um höchstens 80 %, insbesondere um höchstens 60 % größeren Vorspannung fixiert sind als die zweiten Elastifizierungsmittel.

Die ersten Elastifizierungsmittel sind gegenüber den Chassismaterialien mit einer Vorspannung von 3-8, insbesondere 3-7, insbesondere 4 - 7 und weiter insbesondere 4-6 fixiert sind.

Die zweiten Elastifizierungsmittel sind hingegen mit einer demgegenüber geringeren Vorspannung von 2-5, insbesondere 2,5 - 4,5, insbesondere 2,5 - 4 und weiter insbesondere 3 - 4 fixiert.

Der Verbund aus Elastifizierungsmitteln und Chassismaterialien des erfindungsgemäßen Inkontinenzartikels ist vorzugsweise wie folgt ausgebildet: Es wird vorgeschlagen, dass die zweiten Elastifizierungsmittel zwischen einer die körperabgewandte Sichtseite des Bauchabschnitts bzw. des Rückenabschnitts bildenden Chassismateriallage, vorzugsweise aus Vlies, und einer bezüglich dieser innenliegenden Chassismateriallage, vorzugsweise aus Vlies, angeordnet und fixiert sind, wobei die innenliegende Chassismateriallage in Längsrichtung des Inkontinenzartikels im wesentlichen nicht über den zugeordneten Überlappungsbereich von Schrittabschnitt und Bauchabschnitt bzw. Schrittabschnitt und Rückenabschnitt hinaus erstreckt ist. Dies ist der Fall wenn der Überstand höchstens einige mm, insbesondere höchstens 8 mm, vorzugsweise höchstens 5 mm beträgt.

Nach einem weiteren Erfindungsgedanken wird vorgeschlagen, dass die zweiten Elastifizierungsmittel in einem Leimbett zwischen Chassismateriallagen fixiert sind und/oder die ersten Elastifizierungsmittel durch Einzelstrangbeleimung zwischen Chassismateriallagen fixiert sind. Durch die Einzelstrangbeleimung im Bereich der ersten Elastifizierungsmittel lässt sich eine Versteifung des oberen quer elastifizierten hüftseitigen Bereichs des Bauch- und Rückenabschnitts vermeiden. Zudem erweist es sich hierbei als besonders wesentlich und vorteilhaft, dass die aneinander anliegenden Chassismateriallagen dann nicht flächenhaft durchgehend miteinander fixiert sind. Sie können sich außerhalb der einzelstrangbeleimten ersten Elastifizierungsmittel daher voneinander abheben. Im Zusammenwirken mit den ersten Elastifizierungsmitteln ergibt sich so eine optisch ansprechende und zudem sehr weiche und nachgiebige Rüschenbildung. Im Zusammenwirken mit dem größeren Abstand der ersten Elastifizierungsmittel voneinander und einer höheren Rückstellkraft eines jeweiligen ersten Elastifizierungsmittels, die sich durch größere Fadenstärke und/oder größere Vorspannung erreichen lässt, kann ein in Dickenrichtung höherer oder größerer Faltenwurf der Chassismaterialien im Bereich der ersten Elastifizierungsmittel erreicht werden. Auch dies bewirkt eine gute Passform. Es führt aber auch zu einer besseren Griffigkeit oder Greifbarkeit des insgesamt in seiner Höhe reduzierten Bauch- und Hüftbands.

Dadurch, dass die zweiten Elastifizierungsmittel vorzugsweise in einem durchgehenden Leimbett zwischen Chassismateriallagen fixiert sind, ergibt sich ein Laminat, bei dem die Lagen flächenhaft durchgehend verbunden sind. Dies erleichtert das Fügen der Chassismaterialien mit weiteren Bestandteilen, da keine unbestimmten Fältelungen durch nicht miteinander fixierte Materialien gebildet werden. Die Leimbettfixierung der zweiten Elastifizierungsmittel erweist sich daher gerade im Hinblick auf die Fügung des Schrittabschnitts mit dem Bauchabschnitt bzw. mit dem Rückenabschnitt als vorteilhaft. Der vordere und hintere Überlappungsbereich von Schrittabschnitt und Bauchabschnitt bzw. Rückenabschnitt befindet sich nämlich in dem Bereich, in dem die zweiten Elastifizierungsmittel geführt werden.

Weiter erweist es sich als vorteilhaft, wenn die ersten Elastifizierungsmittel im Bauchabschnitt und im Rückenabschnitt durch Einzelstrangbeleimung zwischen einer körperabgewandten Chassismateriallage und einer körperzugewandten Chassismateriallage fixiert sind und die körperzugewandte Chassismateriallage das zugeordnete Längsende des Schrittabschnitts auf dessen körperzugewandter Seite überragt oder überlappt. Auf diese Weise kann ein für den Benutzer unangenehmer Materialübergang zwischen dem Längsende des Schrittabschnitts und dem Bauchabschnitt bzw. Rückenabschnitt vermieden bzw. überfangen werden, so dass Hautreizungen vermieden werden können.

Weiter erweist es sich als vorteilhaft, wenn die ersten Elastifizierungsmittel im Bauchabschnitt und im Rückenabschnitt durch Einzelstrangbeleimung zwischen einer körperabgewandten Chassismateriallage und einer körperzugewandten Chassismateriallage fixiert sind, wobei am Hüftrand keine der Chassismateriallagen um die andere umgefaltet ist, und die körperzugewandte Chassismateriallage und die körperabgewandte Chassismateriallage entlang der Hüftöffnung am Hüftrand nicht miteinander fixiert sind, so dass sie jeweils einen frei ausmündenden hüftseitigen Randabschnitt bilden. Dieser frei ausmündende hüftseitige Randabschnitt entspricht dann gewissermaßen dem Abstand des äußersten hüftzugewandten ersten Elastifizierungsmittels vom geometrischen Rand. Dieser Randabschnitt misst in Längsrichtung vorzugsweise wenigstens 4 mm, insbesondere wenigstens 5 mm, insbesondere höchstens 15 mm, insbesondere höchstens 12 mm und weiter insbesondere höchstens 10 mm. Hierdurch wird die Weichheit des in Umfangsrichtung verlaufenden Hüftrands des Inkontinenzartikels vergrößert, was vom Benutzer als angenehm empfunden wird.

Die chassisbildenden Materialien von Bauchabschnitt und/oder Rückenabschnitt umfassen vorzugsweise Vliesstoffmaterialien, wie Spinnvliese, Kardenvliese oder Through Air bonded Kardenvliese. Besonders bevorzugt umfasst das chassisbildende Material von Bauchabschnitt und/oder Rückenabschnitt ein Spinnvliesmaterial. Die für Bauchabschnitt und/oder Rückenabschnitt eingesetzten Vliesstoffmaterialien haben vorteilhafterweise ein Flächengewicht von 10 - 30 g/m$^2$, weiter vorzugsweise von 15 - 25 g/m$^2$. Besonders bevorzugt umfassen der Bauchabschnitt und der Rückenabschnitt ein Spinnvlies, insbesondere aus Polypropylen, insbesondere mit einem Flächengewicht von 15-25 g/m$^2$. Geringe Flächengewichte der chassisbildenden Materialien von Bauchabschnitt und/oder Rückenabschnitt, insbesondere in Form umfassend oder bestehend aus Vliesstoffmaterialien ermöglichen aufgrund ihrer Flexibilität besonders vorteilhaft die Ausbildung von hautfreundlichen Strukturen.

Der Schrittabschnitt umfasst vorteilhafterweise ein flüssigkeitsundurchlässiges Backsheet-Material und ein Vlies-Topsheet-Material. Das Backsheet umfasst insbesondere eine Folie, insbesondere eines Flächengewichts von 8-20 g/m², insbesondere von 8-16 g/m², insbesondere von 8-14 g/m².

Insbesondere umfasst das Backsheet eine im Gebrauch flüssigkeitsdichte aber gleichwohl atmungsaktive, also wasserdampfdurchlässige, insbesondere mikroporöse Folie.

Der Absorptionskörper umfasst Körperflüssigkeiten absorbierende Materialien wie natürliche oder synthetische Fasern, insbesondere Zellulosefasern, vorzugsweise in Form von Zellstofffluff. Vorzugsweise umfasst der Absorptionskörper außerdem superabsorbierende Materialien (SAP), insbesondere auf Basis oberflächenvernetzter, teilneutralisierter Polyacrylate.

Weiter erweist es sich als vorteilhaft, wenn die Fläche des Überlappungsbereichs von Schrittabschnitt und Bauchabschnitt bezogen auf die Fläche des Bauchabschnitts wenigstens 20 %, insbesondere 20-35 %, insbesondere 20 - 30 % beträgt und/oder wenn die Fläche des Überlappungsbereichs von Schrittabschnitt und Rückenabschnitt bezogen auf die Fläche des Rückenabschnitts wenigstens 25 %, insbesondere 25 - 45 %, insbesondere 25 - 40 %, insbesondere 27 - 40 % beträgt.

Der Anteil der Fläche des Schrittabschnitts an der projizierten Gesamtfläche des ausgedehnten Inkontinenzartikels beträgt vorzugsweise 30 bis 60 %, insbesondere 30 bis 50 %.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Inkontinenzartikels. In der Zeichnung zeigt:

     Figur 1 eine Draufsicht auf einen erfindungsgemäßen Inkontinenzartikel, wobei ein Bauchabschnitt, ein Rückenabschnitt und Schrittabschnitt des Inkontinenzartikels noch nicht zur Bildung einer Höschenform verbunden, sondern in flachgelegtem und eben ausgedehntem Zustand dargestellt sind;

     Figur 2a,b schematische Schnittansichten des Schrittabschnitts im Bereich der Quermittellinie bzw. im Überlappungsbereich von Schrittabschnitt und Rückenabschnitt;

     Figur 3 eine Figur 1 entsprechende Darstellung, wobei die Fixierung des Schrittabschnitts mit dem Bauchabschnitt und dem Rückenabschnitt mittels Klebestreifen erkennbar ist;

     Figur 4 eine vergrößerte ausschnittsweise Darstellung im Bereich des Überlappungsbereichs von Schrittabschnitt und Bauchabschnitt bzw. Schrittabschnitt und Rückenabschnitt des Inkontinenzartikels nach Figur 3;

     Figur 5 eine schematische Schnittansicht der wesentlichen Einzelkomponenten der Chassismaterialien entlang der Längsmittelachse des Inkontinenzartikels;

     Figur 6 eine schematische Ansicht des fertig konfigurierten Inkontinenzartikels

     Figur 7 eine Figur 1 entsprechende Darstellung zur Verdeutlichung von Abmessungen;

     Figur 8 eine Figur 1 entsprechende Darstellung zur Verdeutlichung des Aufbaus des Absorptionskörpers und der Faltachsen;

     Figur 9 eine schematische Längsschnittansicht des Absorptionskörpers entlang der Längsmittelachse;

     Figur 10a,b,c drei schematische Ansichten des Inkontinenzartikels zur Verdeutlichung der Faltung und

     Figur 11 eine schematische Ansicht des gefalteten Inkontinenzartikels zur Verdeutlichung der Probennahme bei der Dickenbestimmung.

Die Figuren zeigen einen insgesamt mit dem Bezugszeichen 2 bezeichneten Inkontinenzartikel in Höschenform für die Aufnahme fester und flüssiger Körperausscheidungen. Der Inkontinenzartikel 2 ist aus drei weitestgehend unabhängig voneinander fertigbaren Komponenten, nämlich einem vorderen Bauchabschnitt 4, einem hinteren Rückenabschnitt 6 und einem zwischen diesen angeordneten und einen Absorptionskörper 7 aufweisenden Schrittabschnitt 8 gebildet, wobei der Schrittabschnitt 8 in einer Längsrichtung 9 des Inkontinenzartikels 2 erstreckt ist und mit einem wesentlichen Flächenanteil des Bauchabschnitts 4 einerseits und des Rückenabschnitts 6 andererseits überlappt und im Überlappungsbereich auf noch näher zu beschreibende Weise herstellerseitig unlösbar verbunden ist. Wie aus Figur 1 ersichtlich, führt dies zu einer H-förmigen Grundstruktur des Inkontinenzartikels. Die in Figur 1 dargestellten miteinander gefügten Bestandteile werden dann zur Bildung der Höschenform an jeweiligen seitlichen Längsrandabschnitten 10, 12 des Bauchabschnitts 4 und des Rückenabschnitts 6 ebenfalls herstellerseitig miteinander durch übliche Fügeverfahren verbunden, wodurch beidseits Seitennahtbereiche 14 ausgebildet werden. In diesem herstellerseitig gefertigten höschenförmigen Zustand des Inkontinenzartikels 2 erstrecken sich der Bauchabschnitt 4 und der Rückenabschnitt 6 in einer Quer- oder Hüftumfangsrichtung 16 durchgehend und definieren so mit ihrem Hüftrand 17 eine in Hüftumfangsrichtung geschlossene Hüftöffnung 18; ferner begrenzen sie zusammen mit dem Schrittabschnitt 8 Beinöffnungen 19, durch welche hindurch der Benutzer den Inkontinenzartikel wie ein Höschen anlegt.

Der Bauchabschnitt 4 lässt sich in einen hüftseitigen Bereich 20 und in einen schrittseitigen und den Beinöffnungen 19 zugewandten Bereich 22 unterteilen. Eine entsprechende Unterteilung ist im Rückenabschnitt 6 vorgesehen, und zwar ebenfalls in einen hüftseitigen Bereich 24 und einen schrittseitigen und den Beinöffnungen 19 zugewandten Bereich 26.

In dem hüftseitigen Bereich 20 des Bauchabschnitts 4 und in dem hüftseitigen Bereich 24 des Rückenabschnitts 6 sind erste Elastifizierungsmittel 28, 29 vorgesehen, bei denen es sich insbesondere um fadenförmige Elastifizierungsmittel, wie Lycra-Fäden, handeln kann, die in vorgedehntem Zustand, im sogenannten Stretch-Bond-Verfahren, mit den Flachmaterialien (Chassismaterialien) des Bauchabschnitts 4 und des Rückenabschnitts 6 verbunden sind. Diese ersten Elastifizierungsmittel 28, 29 erstrecken sich in Quer- oder Hüftumfangsrichtung 16 von einem Seitennahtbereich 14 zum anderen.

Der schrittseitige und den Beinöffnungen 19 zugewandte Abschnitt 22 und 26 des Bauchabschnitts 4 bzw. des Rückenabschnitts 6 haben eine von der Queroder Hüftumfangsrichtung 16 abweichende in Richtung auf eine Quermittelachse 30 des Schrittabschnitts 8 zulaufende Randkontur 32 bzw. 34. Diese Randkontur 32, 34 ist auch in der Darstellung nach Figur 1 bogenförmig und daher zur Begrenzung der Beinöffnungen 19 geeignet.

Durch diesen Verlauf des schrittseitigen und den Beinöffnungen zugewandten Bereichs 22 bzw. 26 wird auch ein verhältnismäßig großer Überlappungsbereich 36, 38 zwischen Schrittabschnitt 8 und Bauchabschnitt 4 bzw. Rückenabschnitt 6 realisiert, der im Hinblick auf eine reißfeste Verbindung von Schrittabschnitt 8 und Bauchabschnitt 4 bzw. Rückenabschnitt 6 wesentlich ist.

Der jeweilige schrittseitige und den Beinöffnungen 19 zugewandte Bereich 22, 26 des Bauchabschnitts 4 bzw. des Rückenabschnitts 6 ist ebenfalls elastifiziert ausgebildet. Dort sind zweite Elastifizierungsmittel 40 bzw. 42 vorgesehen. Die zweiten Elastifizierungsmittel 40, 42 erstrecken sich jeweils ausgehend von den Seitennahtbereichen 14 in Richtung auf eine Längsmittelachse 44 des Inkontinenzartikels. Wie aus Figur 1 zu erkennen ist, fächern die zweiten Elastifizierungsmittel 40, 42 in Richtung auf die Längsmittelachse 44 auf. Dies bedeutet, dass der Abstand zwischen ihnen in Richtung auf die Längsmittelachse 44 zunimmt. Die zweiten Elastifizierungmittel 40, 42 unterqueren den Schrittabschnitt 8. Sie können im Bereich unterhalb des Absorptionskörpers 7 deaktiviert, d.h. ihrer elastifizierenden Wirkung benommen sein.

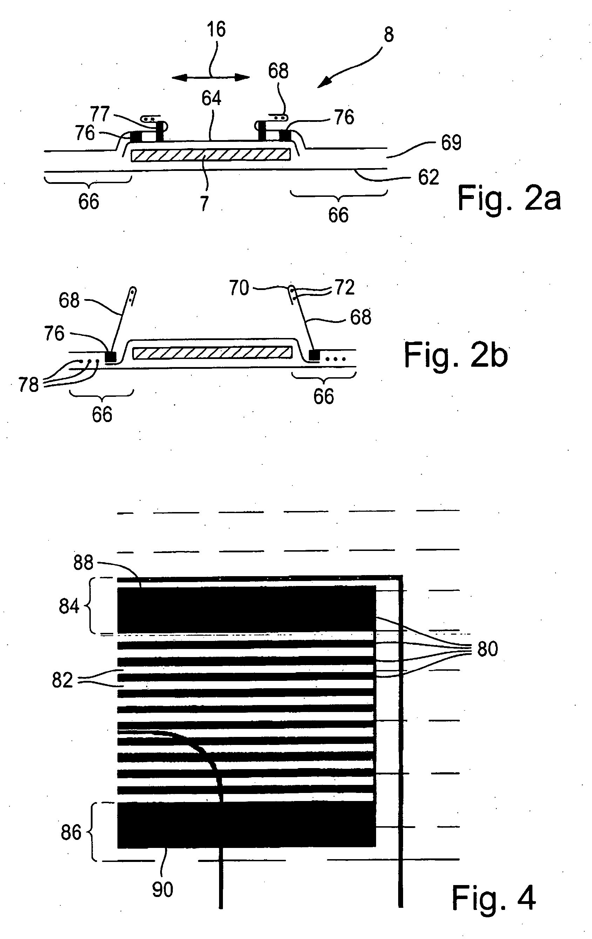

Wie aus Figuren 2a, b ersichtlich umfasst der Schrittabschnitt 8 ein flüssigkeitsundurchlässiges Backsheet-Material 62, das insbesondere von einem atmungsaktiven, jedoch flüssigkeitsdichten Folienmaterial gebildet sein kann, und ein, vorzugsweise auf Vliesbasis beruhendes Topsheet-Material 64. Zwischen dem Backsheet-Material und dem Topsheet-Material ist der Absorptionskörper 7 (nur schematisch dargestellt) angeordnet. Im beispielhaft dargestellten Fall bildet das Backsheet-Material 62 in Querrichtung 16 einen Überhang 66 über den Absorptionskörper 7. Das Topsheet 64 überragt den Absorptionskörper 7 in Querrichtung 16 nur verhältnismäßig geringfügig, und beidseits des Absorptionskörpers 7 ist in Längsrichtung 9 verlaufend jeweils ein aufstehendes Barriéremittel 68 vorgesehen,

welches typischerweise als aufstehendes Cuffelement oder Bündchenelement bezeichnet wird und vorzugsweise aus einem hydrophoben, insbesondere flüssigkeitsundurchlässigen Vliesstoffmaterial gebildet ist, welches sich in Querrichtung 16 vorzugsweise bis zu seitlichen Längsrändern 69 des Schrittabschnitts 8 erstreckt. Die distalen Enden 70 der Barriéremittel 68 sind mit weiteren Elastifizierungsmitteln 72 versehen, welche die Barriéremittel 68 im Gebrauch des Inkontinenzartikels gegen die Hautoberfläche des Benutzers anheben. Die seitlichen Barriéremittel 68 sind über schematisch angedeutete Fixierungen 76, 77 auf das Topsheet 64 beziehungsweise auf sich selbst in einer C-förmig gefalteten Konfiguration festgelegt. Außerhalb des Absorptionskörpers 7 also im Bereich des Überhangs 66 sind ferner Beinelastifizierungsmittel 78 vorgesehen, die vorzugsweise mit einem gewissen Abstand vom materialreichen und damit eher biegesteifen Absorptionskörper 7 verlaufen, um einerseits keine zusätzlichen Dehnungs- oder Verwindungskräfte auf den Absorptionskörper auszuüben, was dessen Absorptionsverhalten nachteilig beeinflussen könnte, und um andererseits einen vom Absorptionskörper weitgehend unbeeinflussten flüssigkeitsdichten Beinabschluss zu realisieren. Diese Beinelastifizierungsmittel 78 enden in Längsrichtung 9 mit einem deutlichen Abstand von insbesondere wenigstens 10 mm, vorzugsweise wenigstens 20 mm vor den zweiten Elastifizierungsmitteln 40 und 42 des Bauchabschnitts 4 beziehungsweise des Rückenabschnitts 6. Vorzugsweise enden diese Beinelastifizierungsmittel 78 in Längsrichtung 9 vor dem Bauchabschnitt 4 und dem Rückenabschnitt 6.

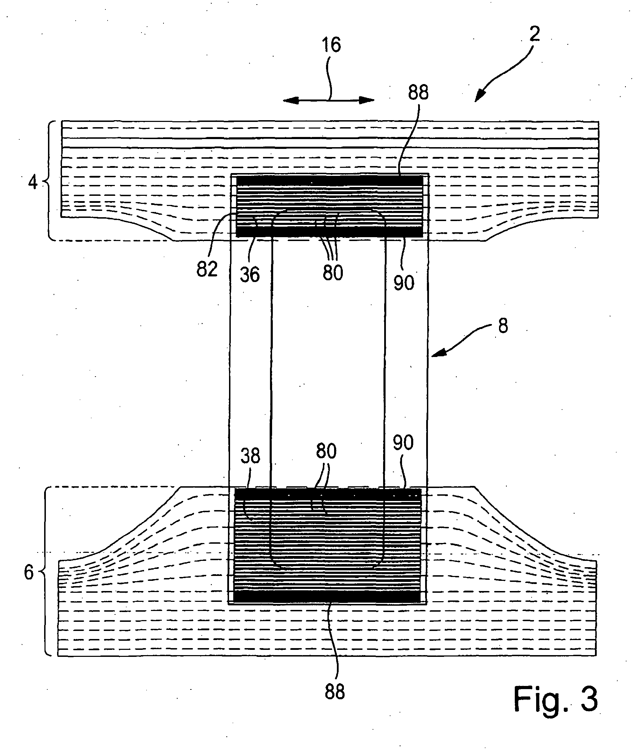

Nachfolgend wird die Fixierung des Schrittabschnitts 8 im vorderen Überlappungsbereich 36 mit dem Bauchabschnitt 4 und im hinteren Überlappungsbereich 38 mit dem Rückenabschnitt 6 beschrieben. Wie anhand der Figuren 3 und 4 ersichtlich, ist hierfür kein vollflächiger Kleberauftrag, sondern eine Vielzahl von im Überlappungsbereich vorgesehener, in Querrichtung 16 erstreckter und parallel zueinander verlaufender Klebestreifen 80 vorgesehen, die durch kleberfreie Streifen 82 voneinander beabstandet sind. Die Klebestreifen 80 erfassen oder überfangen im Wesentlichen den gesamten jeweiligen Überlappungsbereich 36, 38. Im beispielhaft dargestellten, jedoch nicht zwingenden Fall sind in einem in Längsrichtung hüftseitigen Randbereich 84 und in einem in Längsrichtung hüftabgewandten Randbereich 86 des jeweiligen Überlappungsbereichs 36, 38 breitere randseitige Klebestreifen 88 und 90 vorgesehen. Die jeweiligen randseitigen, also hüftzugewandten und hüftfernen Klebestreifen 88, 90 sind mit einer größeren Streifenbreite ausgebildet als die Vielzahl von zwischen ihnen und innen liegenden Klebestreifen 80. Bei einer beispielhaften Ausführungsform beträgt die Breite der randseitigen Klebestreifen 88, 90 quer zu deren Erstreckung 14 mm, die Breite der innen liegenden Klebestreifen 80 2 mm und die Breite der kleberfreien Streifen 82 beträgt 3 mm. Im beispielhaft und bevorzugt dargestellten Fall haben die innen liegenden Klebestreifen 80 vorzugsweise alle dieselbe Breite, und vorzugsweise sind auch die Abstände zwischen ihnen, also die Breite der kleberfreien Streifen 82, gleich. Dessen ungeachtet gelten die eingangs erläuterten Ausführungen zu den Abmessungen und dort beschriebenen Verhältnissen sowie zum Flächengewicht der Kleberbeschichtung bei den Klebestreifen. Auch die Fläche des vorderen und hinteren Überlappungsbereichs 36, 38 bezogen auf die Fläche des Bauchabschnitts 4 bzw. des Rückenabschnitts 6 liegt innerhalb der eingangs erläuterten bevorzugten Grenzen.

Man erkennt des Weiteren aus Figur 3 in Zusammenschau mit Figur 1, dass die zweiten Elastifizierungsmittel 40, 42 im jeweiligen Überlappungsbereich 36, 38 parallel zu den Klebestreifen 80 verlaufen. Auch einige wenige der ersten Elastifizierungsmittel 28 verlaufen im beispielhaft dargestellten Fall im vorderen und hinteren Überlappungsbereich 36, 38 (jedoch auf der körperzugewandten Seite des Schrittabschnitts). Auch die zweiten Elastifizierungsmittel 40, 42 wurden in der Querrichtung 16 durchgehend eingebracht; sie sind im jeweiligen Überlappungsbereich 36, 38 durch eingangs erwähnte Maßnahmen deelastifiziert. Es zeigte sich jedoch, dass die zweiten Elastifizierungsmittel auch im deelastifizierten Zustand - wie eingangs erläutert - erkennbar bleiben. Allerdings werden sie durch die Vielzahl von Klebestreifen 80 kaschiert, und somit wird ihre Erkennbarkeit in erfinderischer Weise reduziert.

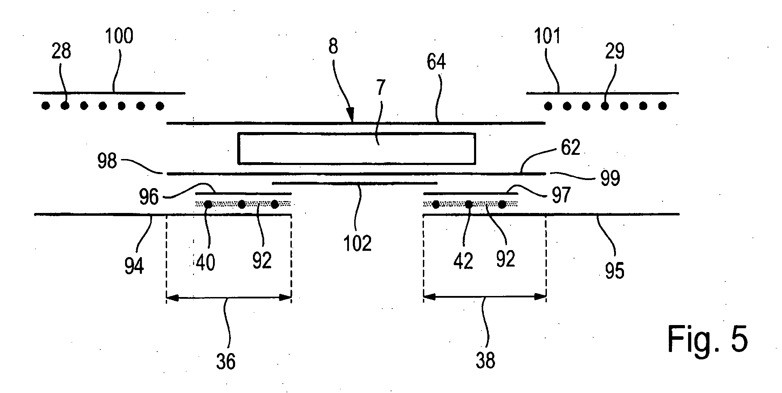

Im bevorzugt dargestellten Fall sind die zweiten Elastifizierungsmittel in einem Leimbett 92 zwischen Chassismateriallagen 94 und 96 bzw. 95 und 97 fixiert (siehe Figur 5). Das Leimbett 92 ist dabei auf eine der Chassismateriallagen 94, 96 bzw. 95, 97 aufgebracht, so dann werden die zweiten Elastifizierungsmittel 40, 42 vorzugsweise endlos auf- bzw. eingelegt und von der weiteren Chassismateriallage überfangen und laminiert. Auf diese Weise werden die zweiten Elastifizierungsmittel 40, 42 fixiert und die Chassismateriallagen 94 und 96 bzw. 95 und 97 flächenhaft durchgehend miteinander gefügt. Bei der körperabgewandten Chassismateriallage 94, 95 handelt es sich um ein atmungsaktives Faservlies, welches der Erstreckung des Bauchabschnitts 4 bzw. Rückenabschnitts 6 entspricht. Bei der Chassismateriallage 96, 97 handelt es sich um ein demgegenüber zurückgesetztes innen liegendes Faservliesmaterial. Es endet in Längsrichtung 9 im bevorzugt dargestellten Fall vor dem Längsende 98, 99 des Schrittabschnitts 8.

Im beispielhaft und bevorzugt dargestellten Fall sind die ersten Elastifizierungsmittel 28, 29 durch Einzelstrangbeleimung zwischen der körperabgewandten Chassismateriallage 94 bzw. 95 und einer weiteren körperzugewandten Chassismateriallage 100, 101 fixiert. Die weitere Chassismateriallage 100, 101 ist wiederum von einem Faservliesmaterial gebildet. Die körperabgewandte und die körperzugewandte Chassismateriallage sind ausschließlich durch die einzelstrangbeleimten ersten Elastifizierungsmittel 28, 29 miteinander verbunden, also nur entlang der Erstreckung dieser ersten Elastifizierungsmittel 28, 29. Die hautfreundlichen Vliesmaterialien sind daher nicht flächenhaft aneinander fixiert, sondern sie können sich voneinander abheben und insbesondere infolge der elastifizierenden Wirkung Fältelungen oder Rüschen bilden. Die körperzugewandte Chassismateriallage 100, 101 erstreckt sich im bevorzugt dargestellten Fall sowohl im Bauchabschnitt 4 als auch im Rückenabschnitt 6 über das jeweilige Längsende 98, 99 des Schrittabschnitts 8 auf dessen körperzugewandter Seite. Es überlappt also diesen Materialübergang und verhindert so eine zu Hautreizungen führende Unstetigkeit.

Man erkennt in Figur 5 des Weiteren, dass das Backsheet 62 des Schrittabschnitts 8 auf seiner körperabgewandten Seite eine Beschichtung 102 aufweist. Diese Beschichtung 102 ist eine Faservliesbeschichtung des im Wesentlichen flüssigkeitsundurchlässigen Backsheets 62. Die Beschichtung 102 erstreckt sich in Längsrichtung 9, jedoch nicht über die gesamte Längserstreckung des Backsheets 62, sondern sie endet verhältnismäßig kurz innerhalb des vorderen und hinteren Überlappungsbereichs 36, 38. Außerhalb des Überlappungsbereichs ist die Beschichtung 102 über die gesamte Erstreckung der körperabgewandten Seite des Backsheets 62 vorgesehen. Die Beschichtung 102 besteht vorzugsweise aus einem Vliesstoff, insbesondere aus einem Spinnvlies, insbesondere aus Polypropylen, insbesondere mit einem Flächengewicht von 10-20 $g/m^2$, insbesondere von 12-17 $g/m^2$.

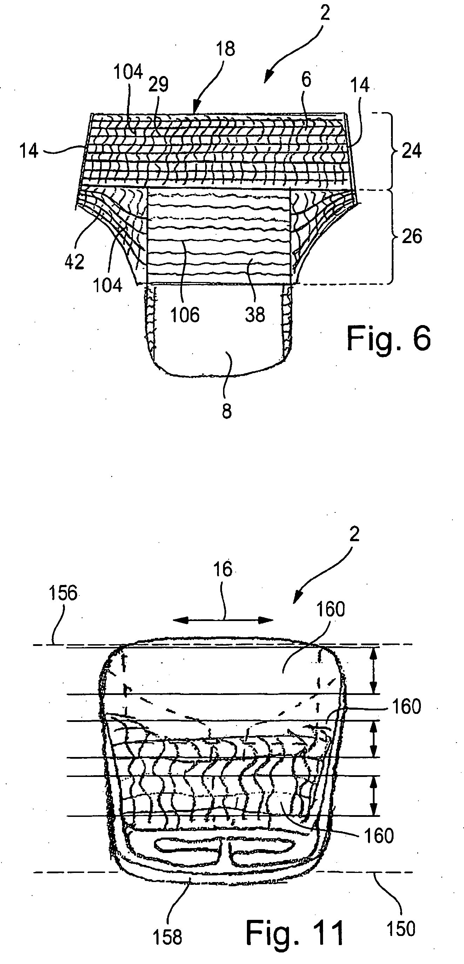

Schließlich zeigt Figur 6 eine schematische Ansicht eines erfindungsgemäßen Inkontinenzartikels im fertig konfigurierten Zustand, in dem der Bauchabschnitt 4 und der Rückenabschnitt 6 durch Ausbildung von Seitennahtbereichen 14 miteinander gefügt sind. Lediglich schematisch angedeutet sind infolge der zusammenziehenden Wirkung der ersten und zweiten Elastifizierungsmittel 28, 29, 40, 42 gebildete Fältelungen oder Rüschen 104, die infolge des Fixierens der Elastifizierungsmittel in vorgedehntem Zustand an den Chassismaterialien (Stretchbondverfahren) gebildet werden. Infolge der Vielzahl von verhältnismäßig feinen Klebestreifen 80 im jeweiligen Überlappungsbereich 36, 38 von Schrittabschnitt 8 und Bauchabschnitt 4 bzw. Rückenabschnitt 6 wird eine visuell und/oder haptisch wahrnehmbare Struktur 106 bei der äußeren Sichtseite des Inkontinenzartikels im jeweiligen Überlappungsbereich 36, 38 gebildet, die hier nur andeutungsweise dargestellt ist. Es wurde erfindungsgemäß festgestellt, dass der streifenförmig aufgebrachte Kleber in die dreidimensional porösen und im Übrigen atmungsaktiv ausgebildeten Faservliesmaterialien, die typischerweise als Chassismaterialien verwendet werden, eindringt und zu einer solchen optisch und/oder haptisch wahrnehmbaren Struktur 106 führt, was sich wie eingangs erläutert als vorteilhaft erweisen kann. Zudem führt die Verbindung von Schrittabschnitt 8 und Bauchabschnitt 4 bzw. Rückenabschnitt 6 durch die Vielzahl von verhältnismäßig schmalen Klebestreifen 80 zu einem sehr wirtschaftlichen Kleberverbrauch, wobei dennoch die erforderlichen Haltekräfte zur sicheren Fügung der drei Komponenten aneinander bereitgestellt werden.

Figur 7 verdeutlicht die Abmessungen, Dimensionen und Verhältnisse eines erfindungsgemäßen Inkontinenzartikels. Man erkennt zunächst, dass die Lage der Quermittelachse 30 die Gesamtlänge des Inkontinenzartikels im flachgelegten Zustand (entsprechend Figur 1) halbiert. Die Quermittelachse 30 bildet auch eine erste in Querrichtung 16 verlaufende Falzachse, um die die Komponenten innerhalb der Herstellungsmaschine gefaltet werden, um die Längsrandabschnitte 10, 12 von Bauchabschnitt 4 und Rückenabschnitt 6 zur Fixierung und Ausbildung von beidseitigen Seitennahtbereichen 14 übereinander zu bringen. Dies findet typischerweise noch unter Führung von endlosen den jeweiligen Bauchabschnitt 4 und Rückenabschnitt 6 bildenden Flachmaterialien, also noch vor der Vereinzelung der Artikel, statt. Man erkennt die Länge L1 zwischen der Quermittelachse 30 und dem jeweiligen Hüftrand 17. Ferner erkennt man die Erstreckung L2 der jeweiligen Seitennaht bzw. des Seitennahtbereichs 14 in Längsrichtung 9, die auch der Länge des jeweiligen Längsrandabschnitts 10 und 12 entspricht. Erfindungsgemäß beträgt das Verhältnis L2/L1 wenigstens 0,42.

Des Weiteren erkennt man den Abstand L4 des äußersten hüftzugewandten ersten Elastifizierungsmittels 28, 29 in Längsrichtung 9 vom innersten schrittzugewandten ersten Elastifizierungsmittel 28, 29. Erfindungsgemäß beträgt das Verhältnis L4/L1 höchstens 0,3.

Man erkennt des Weiteren, dass die ersten Elastifizierungsmittel 28, 29 einen Abstand $d_1$ voneinander haben, der wenigstens 20 % größer ist als der im Seitennahtbereich 14 bestimmte Abstand der zweiten Elastifizierungsmittel 40, 42 voneinander. Im bevorzugt dargestellten Fall haben die ersten Elastifizierungsmittel 28, 29 alle denselben Abstand $d_1$ voneinander, der wenigstens 10 mm, insbesondere 10 bis 15 mm beträgt. Das Verhältnis d1/L4 beträgt bevorzugt 0,08 bis 0,25.

Des Weiteren erkennt man L3 als die Erstreckung des Bauchabschnitts 4 und Rückenabschnitts 6 in Längsrichtung 9, welche für den Bauchabschnitt 4 insbesondere 135 - 260 mm und für den Rückenabschnitt 6 insbesondere 200 - 320 mm beträgt.

Weiter dargestellt ist die Erstreckung Q des Bauchabschnitts 4 bzw. des Rückenabschnitts 6 in Querrichtung 16, die in Verhältnisse L2/Q oder L4/Q eingeht.

Die ersten Elastifizierungsmittel 28, 29 haben eine um wenigstens 20 % größere Fadenstärke als die zweiten Elastifizierungsmittel 40, 42. Sie sind zudem mit einer um wenigstens 10 % größeren Vorspannung mit den Chassismateriallagen im Bauchabschnitt 4 und im Rückenabschnitt 6 fixiert als die zweiten Elastifizierungsmittel 40, 42.

Auf die weiteren bevorzugten eingangs gegebenen Abmessungen, Dimensionen und Verhältnisse wird verwiesen.

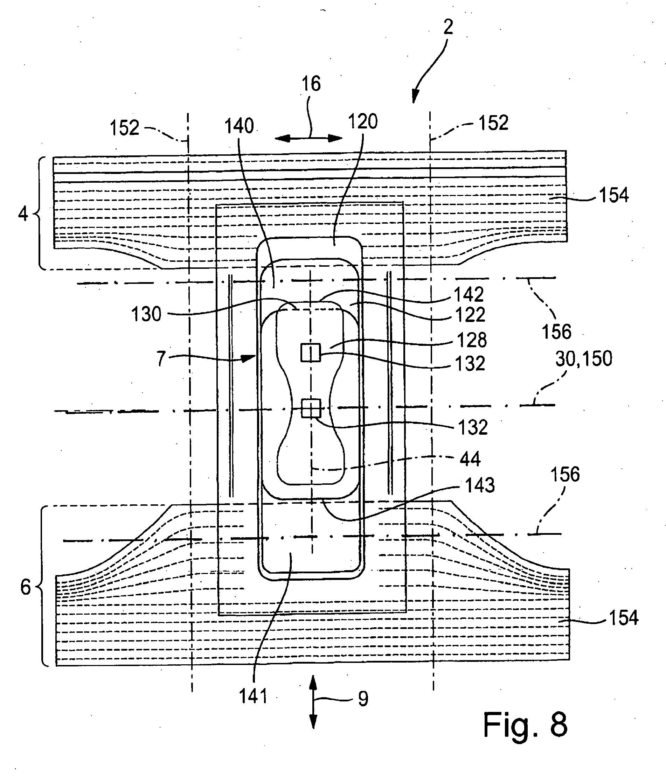

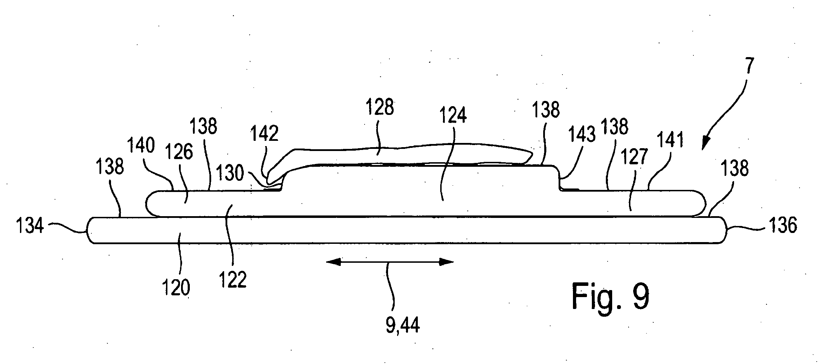

Aus Figuren 8 und 9 ist der Aufbau des Absorptionskörpers 7 in der Draufsicht und in einer Schnittansicht entlang der Längsmittelachse 44 ersichtlich. Der Absorptionskörper 7 umfasst ausgehend von seiner körperabgewandten Seite eine Grundschicht 120 aus zellulosischem Fasermaterial mit einem beispielhaften Flächengewicht von 176 g/m$^2$. Je nach exakter flächenhafter Erstreckung enthält die Grundschicht 10 bis 14 g zellulosisches Fasermaterial.

Auf der Grundschicht 120 ist eine Saugkörperschicht 122 abgelegt, die zumindest hinsichtlich des Flächengewichts an Saugkörpermaterial dreidimensional topologisiert ist. Sie weist in einem mittigen Bereich 124 ein höheres Flächengewicht an Saugkörpermaterial auf als in in Längsrichtung 9 vorderen und hinteren Bereichen 126, 127. Das Flächengewicht an zellulosischem Fasermaterial beträgt in dem vorderen und hinteren Bereich 126, 127 der Saugkörperschicht 122 in dem beispielhaft dargestellten Fall 162 g/m$^2$ und in dem mittigen Bereich 124 329 g/m$^2$. Zusätzlich umfasst die Saugkörperschicht 122 insgesamt etwa 7 g superabsorbierender Polymermaterialien, die homogen gleichförmig in der Saugkörperschicht 122 verteilt angeordnet sind. Die Bereiche 126, 127 und 124 sind in Längsrichtung 9 gegenüber der flächenhaften Erstreckung der Grundschicht 120 zurückgesetzt, wie sich aus Figur 8 ersehen lässt.

Schließlich umfasst der Absorptionskörper 7 eine körperzugewandte im beispielhaft und bevorzugt dargestellten Fall sanduhrförmige Flüssigkeitsaufnahme- und -verteilerschicht 128, die überwiegend auf dem mittleren Bereich 124 der Saugkörperschicht 122 erstreckt ist. Die Flüssigkeitsaufnahme- und -verteilerschicht 128 überragt dabei ein bauchabschnittseitiges Längsende 130 des mittleren Bereichs 124 der Saugkörperschicht 122. Sie umfasst ein Flächengewicht an Fasermaterial, und zwar in Form von intravernetzten Zellulosefasern (curled fiber) von beispielhaft 149 g/m$^2$ mit einer Gesamtmasse von entsprechend der beispielhaften Erstreckung ungefähr 2,8 g.

Die Grundschicht 120, die drei Bereiche 124, 126 und 127 der Saugkörperschicht 122 und die körperzugewandte Flüssigkeitsaufnahme- und -verteilerschicht 128 haben über ihre flächenhafte Erstreckung gleichförmiges Flächengewicht an Saugkörpermaterialien.

Das Flächengewicht wird messtechnisch wie eingangs beschrieben ermittelt, indem ein Prüfling von 25 mm x 25 mm betrachtet wird, der durch sämtliche soeben beschriebene Schichten des Absorptionskörpers 7 ausgestanzt wird. Der auszustanzende Flächenbereich 132 (25 mm x 25 mm) wird immer bezüglich der Längsmittelachse 44 zentriert, so wie dies in Figur 8 angedeutet ist. Wenn das Flächengewicht in Längsrichtung 9 weiter vorn oder weiter hinten bestimmt wird, wird der Prüfling in entsprechender Weise bezüglich der Längsmittelachse 44 zentriert.

Man erkennt, dass das Flächengewicht an Saugkörpermaterial so ausgehend von der Quermittelachse 30 entlang der Längsmittelachse 44 in Richtung auf ein bauchabschnittseitige Ende 134 und in Richtung auf ein rückenabschnittseitige Ende 136 des Absorptionskörpers 7 stufenförmig abnimmt. Auf diese Weise werden zwischen den Stufen Plateaus 138 gebildet. Im Bereich dieser Plateaus 138 ist das Flächengewicht an Saugkörpermaterial der darunter liegenden Schichten des Absorptionskörpers 7 vorzugsweise aber nicht notwendigerweise konstant.

Bei der dargestellten bevorzugten Ausführungsform des Inkontinenzartikels ist das Flächengewicht des Absorptionskörpers 7 ausgehend von der Quermittelachse 30 nach vorn und nach hinten im Bereich der Überlappung der körperzugewandten Flüssigkeitsaufnahme- und

-verteilerschicht 128 mit dem mittigen Bereich 124 der Saugkörperschicht 122 im Wesentlichen konstant.

Man erkennt in Figuren 8 und 9 Plateaus 140, 141, die sich an eine Abstufung 142, 143 in Längsrichtung 9 nach vorn bzw. hinten anschließen. Im Bereich dieser Plateaus 140, 141 ist das Flächengewicht des Absorptionskörpers 7 gegenüber dem Flächengewicht im Bereich der Quermittelachse 30 wesentlich reduziert.

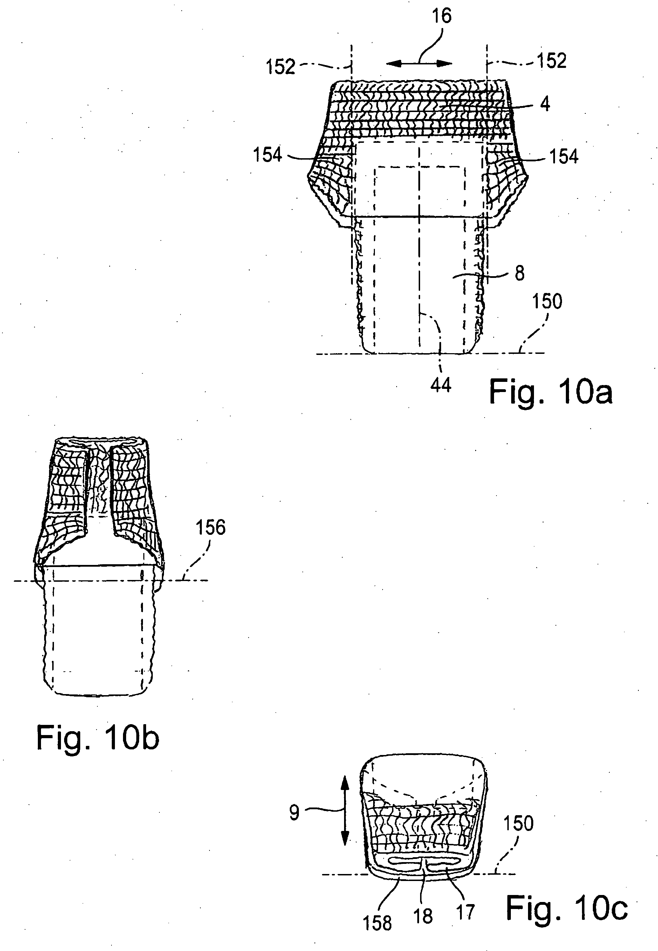

Es wird nun anhand der Figuren 8, 10 und 11 die Faltung des höschenförmigen Inkontinenzartikels für die stapelförmige Anordnung einer Vielzahl von Inkontinenzartikeln in einer Verpackung für die Abgabe in den Handel beschrieben: Wie bereits erwähnt bildet die Quermittelachse 30 eine erste Falzachse 150, um die der Inkontinenzartikel gefaltet wird, so dass Bauchabschnitt 4 und Rückenabschnitt 6 zur Ausbildung von Seitennahtbereichen 14 dauerhaft miteinander gefügt werden können, und zwar durch an sich übliche Fügeverfahren, wie Kleben, Ultraschall, etc.. Des Weiteren sind ungefähr in Längsrichtung 9 erstreckte zweite Falzlinien 152 in Figur 8 lediglich ungefähr angedeutet, da ja die Einfaltung nicht in dem in Figur 8 dargestellten ausgedehnten Zustand erfolgt, sondern nach Fertigstellung des höschenförmigen Inkontinenzartikels in dem in Figur 10a nur schematisch skizzierten Zustand. Ausgehend von diesem in Figur 10a skizzierten Zustand werden beidseitig in Querrichtung 16 seitlich über den Schrittabschnitt 8 überstehende Bereiche 154 des Bauchabschnitts 4 und Rückenabschnitts 6 in Richtung auf die Längsmittelachse 44, und zwar vorzugsweise auf die Außenseite des Bauchabschnitts 4, eingeschlagen, so dass die in Figur 10b ungefähr skizzierte Konfiguration erhalten wird.

Schließlich zeigen die Figuren 8 und 10 eine dritte in Querrichtung 16 verlaufende Falzachse 156, deren Lage bezüglich des Absorptionskörpers 7 aus Figur 8 hervorgeht. Bei weiterer Faltung um diese einzige weitere in Querrichtung 16 erstreckte Falzachse 156 wird die in Figur 10c dargestellte kompakt gefaltete Konfiguration des höschenförmigen Inkontinenzartikels erhalten. Man erkennt, dass der die Hüftöffnung 18 begrenzende Hüftrand 17 in Längsrichtung 9 nicht über die durch die erste Falzachse 150 gebildete äußere Falzkante 158 des Inkontinenzartikels übersteht.

Schließlich verdeutlicht Figur 11, an welchen Stellen die Dicke des insgesamt in die Konfiguration der Figur 10c gefalteten Inkontinenzartikels 2 bestimmt wird. Wie bereits erwähnt, wird der gesamte so gefaltete Inkontinenzartikel 2 über die gesamte Querrichtung 16 mit einem Stanzmesser im Abstand von etwa 10 mm von den Falzkanten bzw. Falzachsen 150 und 156 ausgestanzt, so dass streifenförmige Prüflinge 160 gebildet werden. Anhand dieser sämtliche Lagen des Inkontinenzartikels erfassenden Prüflinge 160 wird dann die Dicke wie eingangs beschrieben ermittelt.

**Patentansprüche**

1. Inkontinenzartikel (2) in Höschenform für die Aufnahme von Körperausscheidungen mit einem vorderen Bauchabschnitt (4) und einem hinteren Rückenabschnitt (6), die von separaten und in einer Längsrichtung (9) entlang einer Längsmittelachse (44) voneinander beabstandeten Komponenten gebildet sind, jedoch zur Bildung eines in Quer- oder Hüftumfangsrichtung (16) durchgehenden Bauch- und Rückenbands mit einer in Hüftumfangsrichtung geschlossenen Hüftöffnung (18) an beidseitigen Seitennahtbereichen (14) herstellerseitig miteinander verbunden sind, wobei jeweils eine in einer Längsrichtung (9) erstreckte Seitennaht gebildet ist, und mit einem einen Absorptionskörper (7) aufweisenden Schrittabschnitt (8), der sich in der Längsrichtung (9) zwischen Bauchabschnitt (4) und Rückenabschnitt (6) erstreckt und mit seiner körperabgewandten Seite an den Bauchabschnitt (4) und an den Rückenabschnitt (6) in einem jeweiligen Überlappungsbereich (36, 38) unlösbar angefügt ist, wobei der Bauchabschnitt (4), der Rückenabschnitt (6) und der Schrittabschnitt (8) gemeinsam Beinöffnungen (19) des Inkontinenzartikels begrenzen, wobei in dem Bauchabschnitt (4) und dem Rückenabschnitt (6) erste Elastifizierungsmittel (28, 29) vorgesehen sind, die sich in einem Abstand voneinander und parallel zueinander in Quer- oder Hüftumfangsrichtung (16) erstrecken und so den Bauchabschnitt (4) und den Rückenabschnitt (6) flächenhaft elastifizieren, wobei in einem schrittseitigen und den Beinöffnungen (19) zugewandten Bereich (22, 26) des Bauchabschnitts (4) und des Rückenabschnitts (6) zweite Elastifizierungsmittel (40, 42) vorgesehen sind, die sich ausgehend von den beiden Seitennahtbereichen (14) in Richtung auf eine Längsmittelachse (44) des Inkontinenzartikels erstrecken und entlang ihrer Erstreckung in Richtung auf die Längsmittelachse (44) des Inkontinenzartikels bogenförmig sich auffächernd mit zunehmendem Abstand voneinander verlaufen und sich bis in den Überlappungsbereich (36, 38) von Schrittabschnitt (8) und Bauchabschnitt (4) bzw. von Schrittabschnitt (8) und Rückenabschnitt (6) erstrecken, wobei sie dort ihrer elastifizierenden Wirkung benommen sein können, insbesondere geschnitten sein können, wobei die Erstreckung (L2) der jeweiligen Seitennaht in Längsrichtung (9) 100 - 170 mm beträgt, **dadurch gekennzeichnet, dass** das Verhältnis (L2/L1) der Erstreckung (L2) der jeweiligen Seitennaht in Längsrichtung (9) zur Erstreckung (L1) des Inkontinenzartikels zwischen Hüftrand (17) und einer Quermittelachse (30) höchstens 0,42 beträgt, und dass im Bauchabschnitt (4) und im Rückenabschnitt (6) das Verhältnis (L4/L1) des Abstands (L4) des äußersten hüftzugewandten ersten Elastifizierungsmittels (28, 29) in Längsrichtung (9) vom innersten schrittzugewandten ersten Elastifizierungsmittel (28, 29) zur Erstreckung (L1) des Inkontinenzartikels zwischen Hüftrand (17) und der Quermittelachse (30) höchstens 0,3 beträgt, und dass die ersten Elastifizierungsmittel (28, 29) einen Abstand voneinander haben, der wenigstens 20 % größer ist als der im Seitennahtbereich (14) bestimmte Abstand der zweiten Elastifizierungsmittel (40, 42) voneinander, und dass die ersten Elastifizierungsmittel (28,29) eine um wenigstens 20 % größere Fadenstärke haben als die zweiten Elastifizierungsmittel (40, 42) und mit einer um wenigstens 10 % größeren Vorspannung im Bauchabschnitt (4) und im Rückenabschnitt (6) fixiert sind als die zweiten Elastifizierungsmittel (40, 42).

2. Inkontinenzartikel nach Anspruch 1, **dadurch gekennzeichnet, dass** das Verhältnis (L2/L1) der Erstreckung (L2) der jeweiligen Seitennaht (14) in Längsrichtung (9) zur Erstreckung (L1) des Inkontinenzartikels zwischen Hüftrand (17) und einer Quermittelachse (30) höchstens 0,4, insbesondere höchstens 0,39, insbesondere höchstens 0,38 und weiter insbesondere wenigstens 0,20, weiter insbesondere wenigstens 0,25, weiter insbesondere wenigstens 0,30 beträgt.

3. Inkontinenzartikel nach Anspruch 1 oder 2, **dadurch gekennzeichnet, dass** im Bauchabschnitt (4) und im Rückenabschnitt (6) das Verhältnis (L4/L1) des Abstands (L4) des äußersten hüftzugewandten ersten Elastifizierungsmittels (28, 29) in Längsrichtung (9) vom innersten schrittzugewandten ersten Elastifizierungsmittel (28, 29) zur Erstreckung (L1) des Inkontinenzartikels zwischen Hüftrand (17) und der Quermittelachse (30) höchstens 0,29 und insbesondere wenigstens 0,12, insbesondere wenigstens 0,15, insbesondere wenigstens 0,18 beträgt.

4. Inkontinenzartikel nach Anspruch 1, 2 oder 3, **dadurch gekennzeichnet, dass** im Bauchabschnitt (4) und im Rückenabschnitt (6) der Abstand (L4) des äußersten hüftzugewandten ersten Elastifizierungsmittels (28, 29) in Längsrichtung (9) vom innersten schrittzugewandten ersten Elastifizierungsmittel (28, 29) höchstens 120 mm, insbesondere höchstens 110 mm, insbesondere höchstens 100 mm, insbesondere höchstens 90 mm und insbesondere wenigstens 60 mm, insbesondere wenigstens 70 mm, insbesondere 70-110 mm, insbesondere 70-100 mm, insbesondere 70 - 90 mm beträgt.

5. Inkontinenzartikel nach einem oder mehreren der vorstehenden Ansprüche, **dadurch gekennzeichnet, dass** die maximale Erstreckung (L3B) des Bauchabschnitts (4) in Längsrichtung (9) 135 - 260 mm, insbesondere 145-250 mm beträgt und/oder die maximale Erstreckung (L3R) des Rückenabschnitts (6) in Längsrichtung (9) 200 - 320 mm, insbesondere 210-310 mm beträgt.

6. Inkontinenzartikel nach einem oder mehreren der vorstehenden Ansprüche, **dadurch gekennzeichnet, dass** ein minimaler Abstand des Bauchabschnitts (4) und des Rückenabschnitts (6) in Längsrichtung (9) voneinander 250 -400 mm, insbesondere 270 - 400 mm, weiter insbesondere 300 - 390 mm beträgt.

7. Inkontinenzartikel nach einem oder mehreren der vorstehenden Ansprüche, **dadurch gekennzeichnet, dass** das Verhältnis (L2/Q) der Erstreckung (L2) der jeweiligen Seitennaht in Längsrichtung (9) zur Erstreckung (Q) des Bauchabschnitts (4) bzw. des Rückenabschnitts (6) in Querrichtung (16) höchstens 0,22, insbesondere höchstens 0,20, insbesondere wenigstens 0,10, insbesondere wenigstens 0,14 beträgt.

8. Inkontinenzartikel nach einem oder mehreren der vorstehenden Ansprüche, **dadurch gekennzeichnet, dass** im Bauchabschnitt (4) und im Rückenabschnitt (6) das Verhältnis (L4/Q) des Abstands (L4) des äußersten hüftzugewandten ersten Elastifizierungsmittels (28, 29) in Längsrichtung (9) vom innersten schrittzugewandten ersten Elastifizierungsmittel (28, 29) zur Erstreckung (Q) des Bauchabschnitts (4) bzw. des Rückenabschnitts (6) in Querrichtung (16) höchstens 0,15, insbesondere höchstens 0,14, insbesondere höchstens 0,13 und insbesondere wenigstens 0,05 und weiter insbesondere wenigstens 0,08 und weiter insbesondere wenigstens 0,10 beträgt.

9. Inkontinenzartikel nach einem oder mehreren der vorstehenden Ansprüche, **dadurch gekennzeichnet, dass** im Bauchabschnitt (4) und/oder im Rückenabschnitt (6) das Verhältnis ($d_1$/L4) des Abstands ($d_1$) der ersten Elastifizierungsmittel (28, 29) in Längsrichtung (9) voneinander zum Abstand (L4) des äußersten hüftzugewandten ersten Elastifizierungsmittels (28, 29) in Längsrichtung (9) vom innersten schrittzugewandten ersten Elastifizierungsmittel (28, 29) zwischen 0,08 und 0,25, insbesondere zwischen 0,09 und 0,20, insbesondere zwischen 0,10 und 0,18 beträgt.

10. Inkontinenzartikel nach einem oder mehreren der vorstehenden Ansprüche, **dadurch gekennzeichnet, dass** der Abstand ($d_1$) der ersten Elastifizierungsmittel (28, 29) in Längsrichtung (9) voneinander wenigstens 8 mm, insbesondere wenigstens 10 mm, insbesondere 10-15 mm, insbesondere 11-14 mm, weiter insbesondere 12-13 mm beträgt.

11. Inkontinenzartikel nach einem oder mehreren der vorstehenden Ansprüche, **dadurch gekennzeichnet, dass** die ersten Elastifizierungsmittel (28, 29) einen Abstand voneinander haben, der wenigstens 30 %, insbesondere wenigstens 50 %, insbesondere höchstens 200 %, insbesondere höchstens 180 %, insbesondere höchstens 150 % größer ist als der im Seitennahtbereich (14) bestimmte Abstand der zweiten Elastifizierungsmittel (40, 42) voneinander.

12. Inkontinenzartikel nach einem oder mehreren der vorstehenden Ansprüche, **dadurch gekennzeichnet, dass** ein minimaler Abstand der zweiten Elastifizierungsmittel (40, 42) voneinander in den Seitennahtbereichen (14) 3-8 mm, insbesondere 3-7 mm, insbesondere 3-6 mm beträgt.

13. Inkontinenzartikel nach einem oder mehreren der vorstehenden Ansprüche, **dadurch gekennzeichnet, dass** die ersten Elastifizierungsmittel (28, 29) eine um wenigstens 30 %, insbesondere um wenigstens 50 %, insbesondere um höchstens 150 %, insbesondere um höchstens 130 %, insbesondere um höchstens 100 % größere Fadenstärke haben als die zweiten Elastifizierungsmittel (40, 42).

14. Inkontinenzartikel nach einem oder mehreren der vorstehenden Ansprüche, **dadurch gekennzeichnet, dass** die Fadenstärke der ersten Elastifizierungsmittel (28, 29) wenigstens 1000 dtex, insbesondere wenigstens 1100 dtex, insbesondere wenigstens 1200 dtex, insbesondere 1200 - 1500 dtex, insbesondere 1200 - 1400 dtex beträgt.

15. Inkontinenzartikel nach einem oder mehreren der vorstehenden Ansprüche, **dadurch gekennzeichnet, dass** die Fadenstärke der zweiten Elastifizierungsmittel (40, 42) 500 - 1100 dtex, insbesondere 600 - 1000 dtex, insbesondere 700 - 900 dtex beträgt.

16. Inkontinenzartikel nach einem oder mehreren der vorstehenden Ansprüche, **dadurch gekennzeichnet, dass** die ersten Elastifizierungsmittel (28, 29) mit einer um wenigstens 20 %, insbesondere um wenigstens 30 %, insbesondere um höchstens 100 %, insbesondere um höchstens 80 %, insbesondere um höchstens 60 % größeren Vorspannung fixiert sind als die zweiten Elastifizierungsmittel (40, 42).

17. Inkontinenzartikel nach einem oder mehreren der vorstehenden Ansprüche, **dadurch gekennzeichnet, dass** die ersten Elastifizierungsmittel (28, 29) mit einer Vorspannung von 3-8, insbesondere 3-7, insbesondere 4-7 und weiter insbesondere 4-6 fixiert sind und/oder dass die zweiten Elastifizierungsmittel (40, 42) mit einer Vorspannung von 2-5, insbesondere 2,5 - 4,5, insbesondere 2,5 - 4 und weiter insbesondere 3-4 fixiert sind.

18. Inkontinenzartikel nach einem oder mehreren der vorstehenden Ansprüche, **dadurch gekennzeichnet, dass** die zweiten Elastifizierungsmittel (40, 42) in einem Leimbett (102) zwischen Chassismateriallagen (94 und 96; 95 und 97) fixiert sind und/oder die ersten Elastifizierungsmittel (28, 29) durch Einzelstrangbeleimung zwischen Chassismateriallagen (94 und 100; 95 und 101) fixiert sind.

19. Inkontinenzartikel nach einem oder mehreren der vorstehenden Ansprüche, **dadurch gekennzeichnet, dass** die ersten Elastifizierungsmittel (28, 29) im Bauchabschnitt (4) und im Rückenabschnitt (6) durch Einzelstrangbeleimung zwischen einer körperabgewandten Chassismateriallage (94, 95) und einer körperzugewandten Chassismateriallage (100, 101) fixiert sind, wobei am Hüftrand (17) keine der Chassismateriallagen (94, 95, 100, 101) um die andere umgefaltet ist, und dass die körperzugewandte Chassismateriallage (100, 101) und die körperabgewandte Chassismateriallage (94, 95) entlang der Hüftöffnung (18) am Hüftrand (17) nicht miteinander fixiert sind, so dass sie jeweils einen frei ausmündenden hüftseitigen Randabschnitt bilden.

**Claims**

1. Incontinence article (2) in pant form for absorbing bodily excretions, with a front stomach section (4) and a rear back section (6), which are formed by separate components that are spaced apart in a longitudinal direction (9) along a longitudinal center axis (44), however, which are connected at lateral seam regions at both sides by the manufacturer for forming a stomach or back band which is continuous in transverse or waist circumferential direction (16) and has a waist opening (18) which is closed in waist circumferential direction, wherein one respective lateral seam is formed which extends in longitudinal direction (9), and with a crotch section (8) which has an absorption body (7) and extends in the longitudinal direction (9) between stomach section (4) and back section (6) and is non detachably attached with its body averted side to the stomach section (4) and the back section (6) in a respective overlapping region (36, 38), wherein the stomach section (4), the back section (6) and the crotch section (8) together delimit leg openings (19) of the incontinence article, wherein in the stomach section (4) and the back section (6) first elastifying means (28, 29) are provided, which extend spaced apart an in parallel relationship to one another in transverse or waist circumferential direction (16) and in this way extensively elastify the stomach section (4) and the back section (6), wherein in a region (22, 26) of the stomach section (4) which is crotch side and faces the leg openings (19), second elastifying means (40, 42) are provided which starting from the two lateral seam regions (14) extend in the direction toward the longitudinal center axis (44) of the incontinence article and thereby fan out arch like with increasing distance to one another and extend as far as into the overlapping region (36, 38) of the crotch section (8) and back section (4) or of the crotch section and stomach section (6), wherein there, they may no longer posses their elastifying effect, and can in particular be cut, wherein the extent (L2) of the respective lateral seam in longitudinal direction (9) is 100 - 170 mm, **characterized in that** the ratio (L2/L1) between the extent (L2) of the respective lateral seam in longitudinal direction (9) and the extent (L1) of the incontinence article between waist border (17) and a transverse center axis (30) is at most 0.42, and **in that** in the stomach section (4) and in the back section (6) the ratio (L4/L1) between the distance (L4) of the outermost waist facing first elastifying means (28, 29) in longitudinal direction (9) to the innermost waist facing first elastifying means (28, 29) and the extent (L1) of the incontinence article between waist border (17) and the transverse center axis (30) is at most 0.3, and **in that** the first elastifying means (28, 29) have a distance to one another which is at least 20% greater than the distance between the second elastifying means (40, 42) to one another defined in the lateral seam region (14), and **in that** the first elastifying means (28, 29) have a thread strength which is greater by at least 20% than a thread strength of the second elastifying means (40, 42) and are fixed in the stomach section (4) and in the back section (6) with a pre-tension which is greater by at least 10% than a pretension of the second elastifying means (40, 42).

2. Incontinence article according to claim 1, **characterized in that** the ratio (L2/L1) between the extend (L2) of the respective lateral seam (14) in longitudinal (9) and the extent (L1) of the incontinence article (9) between waist border (17) and a transverse center axis (30) is at most 0.4, in particular at most 0.39, in particular at most 0.38 and further in particular at least 0.20, further in particular at least 0.25, further in particular at least 0.30.

3. Incontinence article according to claim 1 or 2, **characterized in that** in the stomach section (4) and in the back section (6) the ratio (L4/L1) between the distance (L4) of the outermost waist facing first elastifying means (28, 29) in longitudinal direction (9) from the innermost crotch facing first elastifying means (28, 29) and the extent (L1) of the incontinence article between waist border (17) and the transverse center axis (30) is at most 0.29 and in particular at least 0.12, in particular at least 0.15, in particular at least 0.18.

4. Incontinence article according to claim 1, 2 or 3, **characterized in that** in the stomach section (4) and in the back section (6) the distance (L4) of the outermost waist facing first elastifying means (28, 29) in longitudinal direction (9) to the innermost waist facing first elastifying means (28, 29) is at most 120 mm, in particular at most 110 mm, in particular at most 100 mm, in particular at most 90 mm and in particular at least 60 mm, in particular at least 70 mm, in particular 70 - 110 mm, in particular 70-100 mm, in particular 70 - 90 mm.

5. Incontinence article according to one or more of the preceding claims, **characterized in that** the maximal extent (L3B) of the stomach section (4) in longitudinal direction (9) is 135 - 260 mm, in particular 145 - 250 mm, and/or the maximal extent (L3R) of the back section (6) in longitudinal direction (9) is 200 - 320 mm, in particular 210-310 mm.

6. Incontinence article according to one or more of the preceding claims, **characterized in that** a minimal distance of the stomach section (4) and the back section to each other in longitudinal direction (9) is 250 - 400 mm, in particular 270 - 400 mm, further in particular 300 - 390 mm.

7. Incontinence article according to one or more of the preceding claims, **characterized in that** the ratio (L2/Q) between the extent the extent (L2) of the respective lateral seam in longitudinal direction (9) and the extent (Q) of the stomach section (4) or back section (6) in transverse direction (16) is at most 0.22, in particular at most 0.20, in particular at least 0.10, in particular at least 0.14.

8. Incontinence article according to one or more of the preceding claims, **characterized in that** in the stomach section (4) and in the back section (6) the ratio (L4/Q) between the distance (L4) of the outermost waist facing first elastifying means (28, 29) in longitudinal direction (9) from the innermost crotch facing first elastifying means (28, 29) and the extent (Q) of the stomach section (4) or the back section (6) in transverse direction (16) is at most 0.15, in particular at most 0.14, in particular at most 0.13, and in particular at least 0.05 and further in particular at least 0.08, and further in particular at least 0.10.

9. Incontinence article according to one or more of the preceding claims, **characterized in that** in the stomach section (4) and/or in the back section (6), the ratio (d1/L4) between the distance (d1) of the first elastifying means (28, 29) in longitudinal direction (9) to one another and the distance (L4) of the outermost waist facing first elastifying means (28, 29) in longitudinal direction (9) from the innermost crotch facing first elastifying means (28, 29) is between 0.08 and 0.25, in particular between 0.09 and 0.20, in particular between 0.10 and 0.18.

10. Incontinence article according to one or more of the preceding claims, **characterized in that** the distance (d1) of the first elastifying means (28, 29) in longitudinal direction (9) to each other is at least 8 mm, in particular at least 10 mm, in particular 10-15 mm, in particular 11-14 mm, further in particular 12 - 13 mm.

11. Incontinence article according to one or more of the preceding claims, **characterized in that** the first elastifying means (28, 29) have a distance to one another, which is at least 30%, in particular at least 50%, in particular at most 200%, in particular at most 180 %, in particular at most 150% greater than the distance defined in the lateral seam region (14) of the second elastifying means (40, 42) from each other.

12. Incontinence article according to one or more of the preceding claims, **characterized in that** a minimal distance of the second elastifying means (40, 42) from each other in the lateral seam regions (14) is 3 - 8 mm, in particular 3 - 7 mm, in particular 3- 6 mm.

13. Incontinence article according to one or more of the preceding claims, **characterized in that** the first elastifying means (28, 29) have a thread strength which is greater than a thread strength of the second elastifying means (40, 42) by at least 30%, in particular by at least 50%, in particular by at most 150%, in particular by at most 130 %, in particular by at most 100 %.

14. Incontinence article according to one or more of the preceding claims, **characterized in that** the thread strength of the first elastifying means (28, 29) is at least 1000 dtex, in particular at least 1100 dtex, in particular at least 1200 dtex, in particular 1200 -1500 dtex, in articular 1200 - 1400 dtex.

15. Incontinence article according to one or more of the preceding claims, **characterized in that** the thread strength of the second elastifying means (40, 42) is 500 -1100 dtex, in particular 600 - 1000 dtex, in particular 700 - 900 dtex.

16. Incontinence article according to one or more of the preceding claims, **characterized in that** the first elastifying means (28, 29) are fixed with a pretension that is greater than a pretension of the second elastifying means (40, 42) by at least 30%, in particular by at most 100%, in particular by at most 80%, in particular by at most 60%.

17. Incontinence article according to one or more of the preceding claims, **characterized in that** the first elastifying means (28, 29) is fixed with a pretension of 3- 8, in particular 3-7, in particular 4-7 and further in particular 4-6, and/or that the second elastifying means (40, 42) are fixed with a pretension of 2-5, in particular 2.5 - 4.5, in particular 2.5 - 4 and further in particular 3 - 4.

18. Incontinence article according to one or more of the preceding claims, **characterized in that** the second elastifying means (40, 42) are fixed in a glue bed (102) between chassis material layers (94 and 96; 95 and 97) and/or the first elastifying means (28, 29) are fixed between chassis material layers (94 and 100; 95 and 101) by applying adhesive to individual strands.

19. Incontinence article according to one or more of the preceding claims, **characterized in that** the first elastifying means (28, 29) in the stomach section (4) and the back section (6) are fixed between a body averted chassis material layer (94, 95) and a body facing chassis material layer (100, 101) by applying adhesive to individual strands, wherein on the waist border (17) none of the chassis material layers (94, 95, 100, 101) is folded about the other one, and **in that** the body-facing chassis material layer (100, 101) and the body averted chassis material layer (94, 95) along the waist opening (18) on the waist border (17) are not fixed to one another, so that they respectively form a freely ending waist side border section.

**Revendications**

1. Article d'incontinence (2) sous forme de culotte, destiné à recueillir les excrétions corporelles, comprenant une portion ventrale avant (4) et une portion dorsale arrière (6) qui sont formées par des composants séparés et espacés l'un de l'autre dans une direction longitudinale (9) suivant un axe médian longitudinal (44), mais qui, pour la formation d'une bande ventrale et dorsale continue dans le sens transversal ou dans le sens du tour de hanches (16), avec une ouverture de hanche (18) fermée dans le sens du tour de hanches, sont reliées entre elles par le fabricant au niveau de zones de couture latérale (14) situées de part et d'autre, dans lequel respectivement une couture latérale s'étendant dans une direction longitudinale (9) est formée, et comprenant une portion d'entrejambe (8) présentant un corps absorbant (7), qui s'étend dans la direction longitudinale (9) entre la portion ventrale (4) et la portion dorsale (6) et qui est rapportée de manière inamovible, par sa face montrant dans la direction opposée au corps, sur la portion ventrale (4) et sur la portion dorsale (6) dans une zone de chevauchement (36, 38) respective, dans lequel ladite portion ventrale (4), ladite portion dorsale (6) et ladite portion d'entrejambe (8) délimitent ensemble des ouvertures de jambe (19) de l'article d'incontinence, dans lequel de premiers moyens d'élastification (28, 29) sont prévus dans la portion ventrale (4) et dans la portion dorsale (6), qui s'étendent à distance les uns des autres et parallèlement entre eux dans le sens transversal ou dans le sens du tour de hanches (16) et qui élastifient ainsi en nappe lesdites portions ventrale (4) et dorsale (6), dans lequel de seconds moyens d'élastification (40, 42) sont prévus dans une zone (22, 26) de la portion ventrale (4) et de la portion dorsale (6), qui est située côté entrejambe et montre vers les ouvertures de jambe (19), seconds moyens d'élastification qui s'étendent à partir des deux zones de couture latérale (14) en direction d'un axe médian longitudinal (44) de l'article d'incontinence et qui s'étendent, suivant leur extension en direction de l'axe médian longitudinal (44) de l'article d'incontinence, à distance croissante les uns des autres en arc et en éventail et s'étendent jusque dans la zone de chevauchement (36, 38) des portions d'entrejambe (8) et ventrale (4) ou bien des portions d'entrejambe (8) et dorsale (6), ils y pouvant être dépourvus de leur effet élastifiant, en particulier être coupés, l'extension (L2) de la couture latérale respective dans la direction longitudinale (9) étant comprise entre 100 et 170 mm, **caractérisé par le fait que** le rapport (L2/L1) de l'extension (L2) de la couture latérale respective dans la direction longitudinale (9) à l'extension (L1) de l'article d'incontinence entre le bord de hanche (17) et un axe médian transversal (30) est de 0,42 tout au plus, et que, dans la portion ventrale (4) et dans la portion dorsale (6), le rapport (L4/L1) de la distance (L4), dans la direction longitudinale (9), entre le premier moyen d'élastification (28, 29) le plus extérieur qui montre vers la hanche et le premier moyen d'élastification (28, 29) le plus intérieur qui montre vers l'entrejambe, à l'extension (L1) de l'article d'incontinence entre le bord de hanche (17) et ledit axe médian transversal (30) est de 0,3 tout au plus, et que les premiers moyens d'élastification (28, 29) présentent une distance les uns des autres qui est d'au moins 20 % supérieure à la distance des seconds moyens d'élastification (40, 42) les uns des autres qui est déterminée dans la zone de couture latérale (14), et que les premiers moyens d'élastification (28, 29) présentent une grosseur de fil qui est d'au moins 20 % plus importante que celle des seconds moyens d'élastification (40, 42) et sont fixés, dans les portions ventrale (4) et dorsale (6), avec une précontrainte qui est d'au moins 10 % plus importante que celle des seconds moyens d'élastification (40, 42).

2. Article d'incontinence selon la revendication 1, **caractérisé par le fait que** le rapport (L2/L1) de l'extension (L2) de la couture latérale (14) respective dans la direction longitudinale (9) à l'extension (L1) de l'article d'incontinence entre le bord de hanche (17) et un axe médian transversal (30) est de 0,4 tout au plus, en particulier de 0,39 tout au plus, en particulier de 0,38 tout au plus et encore en particulier d'au moins 0,20, encore en particulier d'au moins 0,25, encore en particulier d'au moins 0,30.

3. Article d'incontinence selon la revendication 1 ou 2, **caractérisé par le fait que**, dans la portion ventrale (4) et dans la portion dorsale (6), le rapport (L4/L1) de la distance (L4), dans la direction longitudinale (9), entre le premier moyen d'élastification (28, 29) le plus extérieur qui montre vers la hanche et le premier moyen d'élastification (28, 29) le plus intérieur qui montre vers l'entrejambe, à l'extension (L1) de l'article d'incontinence entre le bord de hanche (17) et ledit axe médian transversal (30) est de 0,29 tout au plus et en particulier d'au moins 0,12, en particulier d'au moins 0,15, en particulier d'au moins 0,18.

4. Article d'incontinence selon la revendication 1, 2 ou 3, **caractérisé par le fait que**, dans la portion ventrale (4) et dans la portion dorsale (6), la distance (L4), dans la direction longitudinale (9), entre le premier moyen d'élastification (28, 29) le plus extérieur qui montre vers la hanche et le premier moyen d'élastification (28, 29) le plus intérieur qui montre vers l'entrejambe, est de 120 mm tout au plus, en particulier de 110 mm tout au plus, en particulier de 100 mm tout au plus, en particulier de 90 mm tout au plus et en particulier d'au moins 60 mm, en particulier d'au moins 70 mm, en particulier comprise entre 70 et 110 mm, en particulier entre 70 et 100 mm, en particulier entre 70 et 90 mm.

5. Article d'incontinence selon l'une ou plusieurs des revendications précédentes, **caractérisé par le fait que** l'extension maximale (L3B) de la portion ventrale (4) dans la direction longitudinale (9) est comprise entre 135 et 260 mm, en particulier entre 145 et 250 mm et/ou que l'extension maximale (L3R) de la portion dorsale (6) dans la direction longitudinale (9) est comprise entre 200 et 320 mm, en particulier entre 210 et 310 mm.

6. Article d'incontinence selon l'une ou plusieurs des revendications précédentes, **caractérisé par le fait qu'**une distance minimale de la portion ventrale (4) et de la portion dorsale (6) l'une de l'autre dans la direction longitudinale (9) est comprise entre 250 et 400 mm, en particulier entre 270 et 400 mm, encore en particulier entre 300 et 390 mm.

7. Article d'incontinence selon l'une ou plusieurs des revendications précédentes, **caractérisé par le fait que** le rapport (L2/Q) de l'extension (L2) de la couture latérale respective, dans la direction longitudinale (9), à l'extension (Q) de la portion ventrale (4) ou bien de la portion dorsale (6), dans le sens transversal (16), est de 0,22 tout au plus, en particulier de 0,20 tout au plus, en particulier d'au moins 0,10, en particulier d'au moins 0,14.

8. Article d'incontinence selon l'une ou plusieurs des revendications précédentes, **caractérisé par le fait que**, dans la portion ventrale (4) et dans la portion dorsale (6), le rapport (L4/Q) de la distance (L4), dans la direction longitudinale (9), entre le premier moyen d'élastification (28, 29) le plus extérieur qui montre vers la hanche et le premier moyen d'élastification (28, 29) le plus intérieur qui montre vers l'entrejambe, à l'extension (Q) de la portion ventrale (4) ou bien de la portion dorsale (6), dans le sens transversal (16), est de 0,15 tout au plus, en particulier de 0,14 tout au plus, en particulier de 0,13 tout au plus et en particulier d'au moins 0,05 et encore en particulier d'au moins 0,08 et encore en particulier d'au moins 0,10.

9. Article d'incontinence selon l'une ou plusieurs des revendications précédentes, **caractérisé par le fait que**, dans la portion ventrale (4) et/ou dans la portion dorsale (6), le rapport ($d_1$/L4) de la distance ($d_1$) des premiers moyens d'élastification (28, 29) les uns des autres, dans la direction longitudinale (9), à la distance (L4), dans la direction longitudinale (9), entre le premier moyen d'élastification (28, 29) le plus extérieur qui montre vers la hanche et le premier moyen d'élastification (28, 29) le plus intérieur qui montre vers l'entrejambe est compris entre 0,08 et 0,25, en particulier entre 0,09 et 0,20, en particulier entre 0,10 et 0,18.

10. Article d'incontinence selon l'une ou plusieurs des revendications précédentes, **caractérisé par le fait que** la distance ($d_1$) des premiers moyens d'élastification (28, 29) les uns des autres, dans la direction longitudinale (9), est d'au moins 8 mm, en particulier d'au moins 10 mm, en particulier comprise entre 10 et 15 mm, en particulier entre 11 et 14 mm, encore en particulier entre 12 et 13 mm.

11. Article d'incontinence selon l'une ou plusieurs des revendications précédentes, **caractérisé par le fait que** les premiers moyens d'élastification (28, 29) présentent une distance les uns des autres qui est d'au moins 30 %, en particulier d'au moins 50 %, en particulier de 200 % tout au plus, en particulier de 180 % tout au plus, en particulier de 150 % tout au plus, supérieure à la distance des seconds moyens d'élastification (40, 42) les uns des autres, qui est déterminée dans la zone de couture latérale (14).

12. Article d'incontinence selon l'une ou plusieurs des revendications précédentes, **caractérisé par le fait qu'**une distance minimale des seconds moyens d'élastification (40, 42) les uns des autres dans les zones de couture latérale (14) est comprise entre 3 et 8 mm, en particulier entre 3 et 7 mm, en particulier entre 3 et 6 mm.

13. Article d'incontinence selon l'une ou plusieurs des revendications précédentes, **caractérisé par le fait que** les premiers moyens d'élastification (28, 29) présentent une grosseur de fil qui est d'au moins 30 %, en particulier d'au moins 50 %, en particulier de 150 % tout au plus, en particulier de 130 % tout au plus, en particulier de 100 % tout au plus, plus importante que celle des seconds moyens d'élastification (40, 42).

14. Article d'incontinence selon l'une ou plusieurs des revendications précédentes, **caractérisé par le fait que** la grosseur de fil des premiers moyens d'élastification (28, 29) est d'au moins 1000 dtex, en particulier d'au moins 1100 dtex, en particulier d'au moins 1200 dtex, en particulier comprise entre 1200 et 1500 dtex, en particulier entre 1200 et 1400 dtex.

15. Article d'incontinence selon l'une ou plusieurs des revendications précédentes, **caractérisé par le fait que** la grosseur de fil des seconds moyens d'élastification (40, 42) est comprise entre 500 et 1100 dtex, en particulier entre 600 et 1000 dtex, en particulier entre 700 et 900 dtex.

16. Article d'incontinence selon l'une ou plusieurs des revendications précédentes, **caractérisé par le fait que** les premiers moyens d'élastification (28, 29) sont fixés avec une précontrainte qui est d'au moins 20 %, en particulier d'au moins 30 %, en particulier de 100 % tout au plus, en particulier de 80 % tout au plus, en particulier de 60 % tout au plus, plus importante que celle des seconds moyens d'élastification (40, 42).

17. Article d'incontinence selon l'une ou plusieurs des revendications précédentes, **caractérisé par le fait**

**que** les premiers moyens d'élastification (28, 29) sont fixés avec une précontrainte comprise entre 3 et 8, en particulier entre 3 et 7, en particulier entre 4 et 7 et encore en particulier entre 4 et 6, et/ou que les seconds moyens d'élastification (40, 42) sont fixés avec une précontrainte comprise entre 2 et 5, en particulier entre 2,5 et 4,5, en particulier entre 2,5 et 4 et encore en particulier entre 3 et 4.

18. Article d'incontinence selon l'une ou plusieurs des revendications précédentes, **caractérisé par le fait que** les seconds moyens d'élastification (40, 42) sont fixés dans un lit de colle (102) entre des couches de matériau de châssis (94 et 96; 95 et 97) et/ou que les premiers moyens d'élastification (28, 29) sont fixés par collage à brin unique entre des couches de matériau de châssis (94 et 100; 95 et 101).

19. Article d'incontinence selon l'une ou plusieurs des revendications précédentes, **caractérisé par le fait que** les premiers moyens d'élastification (28, 29) dans la portion ventrale (4) et dans la portion dorsale (6) sont fixés par collage à brin unique entre une couche de matériau de châssis (94, 95) opposée au corps et une couche de matériau de châssis (100, 101) montrant vers le corps, dans lequel, sur le bord de hanche (17), aucune des couches de matériau de châssis (94, 95, 100, 101) n'est repliée sur l'autre, et que la couche de matériau de châssis (100, 101) montrant vers le corps et la couche de matériau de châssis (94, 95) opposée au corps ne sont pas fixées entre elles le long de l'ouverture de hanche (18) sur le bord de hanche (17), de sorte qu'elles forment chacune une portion marginale côté hanche qui débouche librement.

Fig. 1

Fig. 2a

Fig. 2b

Fig. 4

Fig. 3

Fig. 5

EP 2 849 695 B1

Fig. 6

Fig. 11

Fig. 7

Fig. 8

Fig. 9

**Fig. 10a**

**Fig. 10b**

**Fig. 10c**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102007055628 A1
- WO 2011098226 A1
- US 20090178755 A1
- US 20050107764 A1
- JP 11036103 A
- US 20060025746 A1
- JP 2006204673 A
- US 20040186453 A1
- JP 2008253289 A
- JP 2007082890 A
- WO 2011105475 A1